(19) Europäisches Patentamt / European Patent Office / Office européen des brevets

(11) **EP 2 673 617 B1**

(12) **FASCICULE DE BREVET EUROPEEN**

(45) Date de publication et mention de la délivrance du brevet:
**20.11.2019 Bulletin 2019/47**

(21) Numéro de dépôt: **12703304.1**

(22) Date de dépôt: **08.02.2012**

(51) Int Cl.:
*G01N 5/02* (2006.01)   *G01N 9/00* (2006.01)
*G01N 15/06* (2006.01)   *G01N 29/02* (2006.01)
*G01N 29/24* (2006.01)   *G01N 33/00* (2006.01)

(86) Numéro de dépôt international:
**PCT/EP2012/052073**

(87) Numéro de publication internationale:
**WO 2012/107457 (16.08.2012 Gazette 2012/33)**

(54) **PROCEDE ET DISPOSITIF DE DETECTION ET D'IDENTIFICATION D'UN ANALYTE PRESENT DANS UN MILIEU GAZEUX**

VERFAHREN UND VORRICHTUNG ZUM NACHWEIS UND ZUR IDENTIFIZIERUNG EINES ANALYTEN IN EINEM GASFÖRMIGEN MEDIUM

METHOD AND APPARATUS FOR DETECTING AND IDENTIFYING AN ANALYTE PRESENT IN A GASEOUS MEDIUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.02.2011 FR 1150982**

(43) Date de publication de la demande:
**18.12.2013 Bulletin 2013/51**

(73) Titulaire: **Commissariat à l'Énergie Atomique et aux Énergies Alternatives 75015 Paris (FR)**

(72) Inventeurs:
- **MONTMEAT, Pierre**
  **F-37520 La Riche (FR)**
- **GUILLEMOT, Marianne**
  **F-54520 Laxou (FR)**
- **PRENE, Philippe**
  **75003 PARIS (FR)**
- **LARUE, Anthony**
  **F-92370 Chaville (FR)**
- **SUARD, Frédéric**
  **F-78000 Versailles (FR)**
- **PAUL, Nicolas**
  **F-75010 Paris (FR)**
- **SCHULTZ, Emmanuelle**
  **38120 SAINT-EGREVE (FR)**
- **BORDY, Thomas**
  **F-38600 Fontaine (FR)**
- **ROUSIER, Rodrigue**
  **F-38000 Grenoble (FR)**

(74) Mandataire: **Ahner, Philippe BREVALEX 95, rue d'Amsterdam 75378 Paris Cedex 8 (FR)**

(56) Documents cités:
US-A1- 2002 178 787   US-A1- 2004 042 933
US-A1- 2004 181 346   US-A1- 2004 259 267
US-A1- 2010 259 254   US-B1- 6 955 787
US-B2- 7 446 870

- PENZA M ET AL: "Carbon nanotubes-coated multi-transducing sensors for VOCs detection", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 111-112, 11 novembre 2005 (2005-11-11), pages 171-180, XP027810875, ISSN: 0925-4005 [extrait le 2005-11-11]

**Description**

**DOMAINE TECHNIQUE**

La présente invention se rapporte à un procédé permettant de détecter et d'identifier un analyte présent dans un milieu gazeux.

Un tel procédé est utile pour la détection et l'identification d'explosifs, que ce soit en vue d'assurer la sécurité de lieux publics comme les aéroports, de contrôler la licéité de marchandises en circulation sur un territoire, de lutter contre le terrorisme, de procéder à des opérations de désarmement, de localiser des mines antipersonnel ou encore de dépolluer des sites industriels ou militaires.

Il est également utile pour la protection de l'environnement, en particulier pour le contrôle et la surveillance de la pollution atmosphérique et de la qualité d'ambiances plus ou moins confinées, ainsi que dans la surveillance, à des fins sécuritaires, de sites industriels fabriquant, stockant et/ou manipulant des composés nitrés, comme par exemple des composés nitroaromatiques tels que le nitrobenzène (NB), le dinitrobenzène (DNB), le trinitrobenzène (TNB), le nitrotoluène (NT), le dinitrotoluène (DNT), le 2,4,6-trinitrotoluène (TNT) et analogues.

**ÉTAT DE LA TECHNIQUE ANTÉRIEURE**

La détection d'analytes, en particulier d'analytes présents sous forme de traces (c'est-à-dire présentes en des quantités de l'ordre du picogramme au nanogramme), est particulièrement utile pour la détection de molécules qui sont toxiques ou dangereuses (explosifs, polluants, ...). On entend par « analyte », un composé que l'on souhaite détecter dans un milieu donné comme, par exemple, une atmosphère gazeuse, de l'eau, un sol, etc.

En ce qui concerne la détection d'explosifs, plusieurs méthodes sont utilisées, à l'heure actuelle, pour détecter des vapeurs de composés nitrés entrant dans la constitution des explosifs, comme l'emploi de chiens *"renifleurs"* dressés et entraînés à cet effet, l'analyse en laboratoire d'échantillons prélevés sur site (par exemple par chromatographie couplée à un spectromètre de masse ou à un détecteur à capture d'électrons) ou encore la détection infrarouge.

Ces méthodes font, d'une manière générale, preuve d'une grande sensibilité, ce qui est primordial en matière de détection d'explosifs compte tenu de la très faible concentration en vapeurs de composés nitrés qui règne au voisinage d'un explosif. Elles ne donnent toutefois pas totalement satisfaction.

Ainsi, l'utilisation de chiens *"renifleurs"* présente l'inconvénient de nécessiter une longue formation des chiens et de leurs maîtres et d'être inadaptée à des opérations prolongées en raison de ce que la durée d'attention des chiens est limitée.

Quant aux autres méthodes, l'encombrement des appareillages qu'elles utilisent, leur consommation d'énergie et leurs coûts de mise en œuvre s'opposent au développement de systèmes de détection aisément transportables et autonomes et, partant, aptes à être utilisés sur tout type de sites.

Depuis quelques années, le développement de capteurs chimiques capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique est modifiée au contact des molécules gazeuses recherchées (également appelées molécules cibles), qui revêt un transducteur qui mesure, en temps réel, toute variation de cette propriété physique et permet de mettre ainsi en évidence la présence des molécules gazeuses recherchées. Il est ainsi possible de mesurer des variations de propriétés électriques, magnétiques, optiques, fréquentielles, viscoélastiques, physico-chimiques, de densité, etc, au moyen de transducteurs adaptés tels que les microbalances à quartz (MBQ), les micro-leviers, les dispositifs de fluorescence ou de mesure d'absorption ou de réflexion optiques (colorimètres), les systèmes d'ondes acoustiques de surface (SAW pour Surface Acoustic Wave en anglais), les cellules électrochimiques ou encore les capteurs chimiques semi-conducteurs à base d'oxydes métalliques.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Cependant, quel que soit le transducteur employé, le capteur ainsi formé n'est pas suffisamment spécifique pour éviter les fausses alarmes et permettre de déterminer la nature d'un composé chimique présent à l'état de traces dans l'atmosphère.

Certains auteurs (documents **[1]**, **[2]** et **[3]**) ont proposé de multiplier le nombre de capteurs chimiques présents sur un même transducteur, afin de diversifier les réponses des capteurs en jouant sur la nature des matériaux sensibles déposés sur le transducteur choisi (en l'occurrence, un micro-levier dans le document **[1]** et un capteur semi-conducteur dans les documents **[2]** et **[3]**) et ainsi, de déterminer la nature des composés détectés.

Cependant, outre le fait qu'aucun exemple précis ne soit donné pour la détection d'explosifs, les performances des solutions techniques proposées dans ces documents **[1]** à **[3]** restent limitées par les propriétés intrinsèques de détection de chaque transducteur employé. De plus, les réponses obtenues aux différents gaz, présentées en particulier dans le document **[2],** sont très proches, ce qui révèle une sélectivité réduite des dispositifs de détection, pouvant provoquer des fausses alarmes et/ou des erreurs sur la détermination de la nature du composé chimique présent dans le gaz à analyser.

Par ailleurs, il a été montré que la multiplication de capteurs chimiques issus d'une même technologie dans un dispositif de détection n'est pas suffisante pour en améliorer les performances (documents **[4]** et **[5]**).

D'autres auteurs ont proposé d'utiliser des multicapteurs multitransducteurs pour la détection d'analytes tels que les molécules organiques volatiles (VOC), les produits chimiques industriels nocifs ou encore les agents chimiques de guerre et pour la détermination de leur nature chimique (documents **[6]** et

**[0016]** **[7]**). A titre d'exemple, dans le document **[6],** le dispositif de détection proposé repose sur la mise en parallèle de plusieurs microbalances à quartz (MBQ) et de capteurs chimiques résistifs fournissant des réponses relatives, respectivement, à la masse d'analyte déposée à la surface du matériau sensible et à la variation de volume associée. A partir de ces deux données, il est possible de déterminer la densité de l'analyte détecté et, par voie de conséquence, de connaître la nature chimique de l'analyte, puisque la densité est caractéristique de la nature chimique de l'analyte.

Cependant, l'utilisation d'un tel dispositif de détection pour déterminer la nature chimique d'un composé présent dans l'atmosphère paraît inadaptée car :

- la température a un effet important sur la densité des molécules et cet effet est difficile à estimer ;
- la précision de la mesure ne permet pas de distinguer deux analytes ayant des densités proches ;
- les interférents présents dans l'atmosphère, et en particulier l'humidité, peuvent s'adsorber à la surface du matériau sensible et ainsi altérer la détermination de la densité mesurée.

Dans tous les cas, ces variations de la densité apparente mesurée peuvent entrainer de fausses alarmes et tromper l'utilisateur sur la nature de la molécule détectée.

Dans le document **[7],** le dispositif de détection proposé nécessite l'utilisation d'un détecteur par photo-ionisation (PID) et d'un spectromètre à mobilité ionique (IMS). Or, un spectromètre à mobilité ionique n'est pas performant pour la détection de traces sous forme de gaz. De plus, l'emploi d'un spectromètre à mobilité ionique nécessite la présence d'une source radioactive, ce qui est très contraignant. Un tel dispositif ne permet pas d'envisager la détection, dans l'atmosphère, de traces d'explosifs, qui se caractérisent par de très faibles pressions de vapeur à température ambiante (c'est-à-dire inférieures ou égales à $10^{-3}$ mbar ou 1 ppm à 20°C).

PENZA M et al, "Carbon nanotubes-coated multi-transducing sensors for VOCs detection", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 111-112,2005, pages 171-180, divulguent un procédé de détection et d'identification d'un analyte présent dans un milieu gazeux contenant un ou plusieurs analytes, dans lequel :on utilise un détecteur qui comprend au moins trois capteurs C1, C2 et C3, comprenant chacun un transducteur associé à un matériau sensible capable d'adsorber un ou plusieurs analytes,Cl comprend un transducteur optique tandis que C2 et C3 comprennent des transducteurs gravimétriques différents l'un de l'autre, chacun des capteurs C1, C2 et C3 émet un signal en l'absence de ce ou ces analytes,et l'adsorption dudit ou desdits analytes par le matériau sensible d'un capteur produisant une variation du signal émis par ce capteur ; le procédé comprenant :a) la mise en contact du détecteur avec le milieu gazeux.

US 2004/259267 A1, US 2004/042933 A1, US 2002/178787 A1, US 6 955 787 B1, US 2004/181346 A1, US 2010/259254 A1 et US 7 446 870 B2 divulguent aussi des procédés de détection et d'identification d'un analyte présent dans un milieu gazeux contenant un ou plusieurs analytes.

Compte tenu de ce qui précède, les Inventeurs se sont donc fixé pour but de concevoir un procédé qui permette de détecter et d'identifier avec précision une ou plusieurs molécules présentes sous forme de traces dans un milieu gazeux, comme par exemple dans l'atmosphère et qui, d'une manière générale, soit exempt des différents inconvénients présentés par les procédés proposés à ce jour pour la détection de molécules présentes dans un milieu gazeux.

**EXPOSÉ DE L'INVENTION**

Ce but et d'autres encore sont atteints par la présente invention qui a, en premier lieu, pour objet un procédé de détection et d'identification d'un analyte présent dans un milieu gazeux contenant un ou plusieurs analytes, dans lequel :

on utilise un détecteur qui comprend au moins trois capteurs C1, C2 et C3, comprenant chacun un transducteur associé à un matériau sensible capable d'adsorber un ou plusieurs analytes,
C1 comprend un transducteur optique tandis que C2 et C3 comprennent des transducteurs gravimétriques différents l'un de l'autre,
chacun des capteurs C1, C2 et C3 émet un signal en l'absence de ce ou ces analytes,

et l'adsorption dudit ou desdits analytes par le matériau sensible d'un capteur produisant une variation du signal émis par ce capteur ;

le procédé comprenant :

a) la mise en contact du détecteur avec le milieu gazeux ; et

b) l'acquisition et l'analyse, de manière synchrone et répétitive, des signaux émis par C1, C2 et C3 pendant cette mise en contact pour obtenir, pour chaque capteur, une courbe représentant le signal émis par ce capteur en fonction du temps, cette courbe représentant soit la ligne de base du capteur dans le cas où le matériau sensible de ce capteur n'adsorbe aucun des analytes, soit une cinétique d'adsorption qui est caractéristique du nombre d'analyte(s) adsorbé(s) par le matériau sensible du capteur et de ce(s) analyte(s) ;

moyennant quoi :

si, au cours de l'étape b), une variation du signal émis par l'un au moins de C1, C2 et C3 au-delà d'au moins un seuil S, en valeur absolue, préalablement fixé pour le capteur émettant ce signal est détectée, alors on poursuit les étapes a) et b) et on identifie le ou les analytes adsorbés par le matériau sensible de ce capteur par comparaison de la cinétique d'adsorption obtenue pour ce capteur avec une pluralité de modèles de cinétiques d'adsorption préalablement établies pour chacun des analytes susceptibles d'être détectés par ce capteur.

Selon une première possibilité, la variation du signal détectée comprend une variation instantanée entre deux acquisitions du capteur successives.

Selon une deuxième possibilité, la variation du signal détectée comprend une variation moyenne sur au moins trois acquisitions du capteur successives.

Avantageusement, selon la première et/ou la deuxième possibilité, la détection d'une variation d'un signal au cours de l'étape b) comprend la réalisation, pour chaque capteur C1, C2 et C3, d'un calcul d'extrapolation linéaire d'une valeur extrapolée du $n^{ième}$ signal émis par ce capteur à partir des $(n-1)$ signaux précédemment émis par ce capteur, $n$ étant un nombre entier supérieur ou égal à 3, la variation instantanée étant la différence en valeur absolue entre la valeur extrapolée et la vapeur acquise du $n^{ième}$ signal émis par ledit capteur, la détection du signal étant prise en compte lorsque la valeur, en valeur absolue, de la variation instantanée est supérieure ou égale à une valeur du seuil S1 préalablement fixé.

Avantageusement, selon les possibilités ci-dessus, la détection d'une variation d'un signal au cours de l'étape b) comprend la réalisation, pour chaque capteur C1, C2 et C3, d'un calcul d'une pente à partir des $(n-1)$ signaux précédemment émis par le capteur, $n$ étant un nombre entier supérieur ou égal à 3, la valeur absolue de cette pente correspondant à la variation moyenne, la détection étant prise en compte lorsque la valeur de la variation moyenne est supérieure à une valeur seuil S2 prédéterminée.

Avantageusement, l'identification est obtenue en réalisant les étapes successives suivantes :

- pour chaque capteur et pour chaque modèle, calcul d'une courbe d'interpolation selon ledit modèle à partir des signaux dudit capteur acquis entre un temps $t_D$, correspondant au temps pour lequel une variation de signal émis par l'un au moins des capteurs C1, C2 et C3 est détectée, et un temps t ;
- calcul d'erreurs pour chaque capteur et chaque modèle entre la courbe d'interpolation selon ledit modèle et les signaux dudit capteur acquis entre le temps $t_D$ et le temps t ;
- calcul, pour chaque modèle, d'une probabilité de présence au temps t de l'analyte correspondant audit modèle à partir du calcul d'erreurs obtenu pour chaque capteur.

Selon un mode de réalisation particulier, un coefficient de confiance est attribué à chaque capteur C1, C2 et C3, chacun de ces coefficients reflétant la capacité dudit capteur à retransmettre, avec exactitude, l'adsorption ou la non adsorption d'un analyte sur le matériau sensible du capteur.

Avantageusement, on calcule une différence, pour chaque capteur, entre une valeur de probabilité de présence calculée à un temps t et une valeur de probabilité de présence calculée à un temps t précédent pour ledit capteur et

- si cette différence est inférieure, pour chaque capteur, à une valeur seuil P prédéterminée, alors on compare les valeurs de probabilité avec une valeur seuil D prédéterminée de probabilité et si la différence est supérieure à la valeur seuil prédéterminée P, alors l'analyte du modèle ayant la probabilité la plus importante correspond à l'analyte adsorbé sur le capteur, sinon la détection est considérée comme étant une erreur de détection ;
- si la différence est supérieure à la valeur seuil D prédéterminée, alors on continue la comparaison.

Le calcul d'erreurs peut, par exemple, consister en un calcul d'erreurs quadratiques.

En terme de détection, les capteurs chimiques de gaz faisant appel à des transducteurs gravimétriques ou à des transducteurs optiques constituent des éléments de choix pour détecter des molécules cibles à très basse pression de vapeur comme les composé explosifs, du fait de leurs grandes sensibilité, sélectivité et robustesse en regard des conditions environnementales. En utilisant un détecteur ayant à la fois un capteur à base d'un transducteur optique, un capteur à base d'un premier transducteur gravimétrique et un capteur à base d'un second transducteur gravimétrique, différent du premier, on combine donc les avantages de ces différents capteurs.

De préférence, le transducteur optique est un transducteur à fluorescence, tandis que les transducteurs gravimétriques sont respectivement une microbalance à quartz (MBQ en anglais) et un transducteur à ondes acoustiques (SAW en anglais).

Pour rappel, un capteur gravimétrique comprend, schématiquement, un substrat piézo-électrique (ou résonateur), généralement un cristal de quartz recouvert sur ses deux faces d'une couche métallique, par exemple d'or ou de platine, servant d'électrode. Le matériau sensible recouvrant l'une ou les deux faces du substrat, toute variation de masse de ce matériau se traduit par une variation de la fréquence de vibration du substrat.

Un capteur optique à fluorescence, quant à lui, comprend généralement un substrat en verre ou en quartz de qualité optique dont l'une des faces est recouverte d'un film mince du matériau sensible. L'intensité de la fluorescence émise par le matériau sensible peut être mesurée sur l'ensemble du spectre d'émission de ce matériau. Toutefois, il est préférable d'effectuer les mesures d'intensité de fluorescence à la longueur d'onde d'émission donnant les valeurs d'intensité maximales pour la longueur d'onde d'excitation conduisant, elle, au meilleur rapport signal/bruit pour l'acquisition des intensités de fluorescence.

Selon un mode de réalisation particulier, le détecteur comprend, outre les capteurs C1, C2 et C3, un ou plusieurs capteurs supplémentaires réalisés en plaçant un ou plusieurs matériaux sensibles supplémentaires sur un ou plusieurs des transducteurs des capteurs C1, C2 et C3. Ainsi, au moins un des transducteurs C1, C2 et C3 comprend plus d'un matériau sensible, formant de ce fait plus d'un capteur à partir d'un même transducteur.

Ainsi, il est tout à fait possible de disposer plus d'une couche sensible (d'un même matériau ou de matériaux différents) sur un même transducteur, formant ainsi ce qu'on appelle un multicapteur.

Il est également possible de combiner ou d'adjoindre aux trois capteurs précédemment cités, des capteurs chimiques traditionnels comme les cellules électrochimiques, les micro-leviers ou encore les systèmes résistifs, en particulier pour la détection et la détermination de familles de composés chimiques aux pressions de vapeur plus importantes, comme les composés organiques volatils (VOC) dans le cadre de la protection de l'environnement, en particulier pour le contrôle et la surveillance de la pollution atmosphérique et de la qualité d'ambiances, ainsi que pour la surveillance à des fins sécuritaires, de sites industriels.

Par mesure de comparaison, les performances globales de chaque transducteur sélectionné, MBQ, SAW et fluorescence, pris individuellement en fonction des critères classiques de détection et en vue de la détermination de la nature chimique de traces de molécules chimiques présentes dans l'atmosphère sont regroupées dans le tableau ci-dessous :

| Critères | MBQ | SAW | Fluorescence |
|---|---|---|---|
| **Sensibilité** | - | + | + |
| **Sélectivité** | - | - | ≈ |
| **Réversibilité** | + | + | ≈ |
| **Panel de molécules cibles détectables** | + | + | - |
| **Robustesse aux conditions environnementales** | + | - | + |
| **Temps de réponse** | ≈ | + | - |
| + satisfaisant<br>≈ suffisant<br>- insuffisant | | | |

On constate qu'aucun des transducteurs sélectionnés (MBQ, SAW et fluorescence), qu'ils soient associés à un ou plusieurs matériaux sensibles les plus performants, ne peut permettre, à lui seul, de détecter et de déterminer avec précision la nature chimique du composé chimique présent dans l'atmosphère, du fait du manque de sélectivité des transducteurs. D'ailleurs, ce dernier point peut être à l'origine de fausses alarmes et il est généralement compensé par un nombre élevé de points de données afin de fiabiliser ces dispositifs de détection. Cette acquisition d'un nombre élevé de points de données durant la mesure nécessite du temps, ce qui fait que ce type de capteurs chimiques de gaz se caractérise par une durée de détection relativement longue (typiquement plusieurs minutes). Ceci est dommageable dans les domaines d'application comme la détection d'explosifs dans lesquels la durée de détection constitue un critère primordial de performance pour les équipes assurant, en particulier, la sécurité des lieux publics. De plus, il est couramment admis que des durées de détection de plusieurs minutes constituent un frein pour l'utilisation de tels capteurs chimiques.

Le procédé selon l'invention ne présente pas ces inconvénients. En effet, en combinant différents transducteurs, il est possible de tirer avantage de chacun des transducteurs, aussi bien sur le plan de la sensibilité et de la sélectivité, qu'au niveau de la robustesse aux conditions environnementales. En outre, l'emploi d'un traitement du signal spécifique dont la discrimination repose sur les différences de cinétiques d'adsorption des molécules à détecter sur la surface des différents capteurs chimiques mis en parallèle permet de raccourcir notablement le temps de réponse du détecteur et de déterminer rapidement la nature du gaz présent dans l'atmosphère. L'analyse est réalisée en ligne durant les mesures sur les différents capteurs en parallèle et permet de ce fait de minimiser le temps de réponse du détecteur.

Avantageusement, la détection d'une variation du signal émis par l'un au moins des capteurs C1, C2 et C3 au-delà d'un seuil préalablement fixé pour le capteur émettant ce signal déclenche l'activation d'une alarme, sonore et/ou visuelle, qui avertit un utilisateur du détecteur de la présence d'un ou plusieurs analyte dans le milieu gazeux.

De préférence, le procédé de détection et d'identification selon l'invention est utilisé pour détecter et identifier un ou plusieurs explosifs présent(s) dans un milieu gazeux. Il est particulièrement adapté à la détection et à l'identification de molécules cibles ayant de très basses pressions de vapeur comme c'est le cas des composés explosifs.

L'invention a également pour objet un dispositif de détection et d'identification d'un analyte présent dans un milieu gazeux contenant un ou plusieurs analytes, caractérisé en ce qu'il comprend :

- un détecteur qui comprend au moins trois capteurs C1, C2 et C3, comprenant chacun un transducteur associé à un matériau sensible capable d'adsorber un ou plusieurs analytes,
C1 comprend un transducteur optique tandis que C2 et C3 comprennent des transducteurs gravimétriques différents l'un de l'autre,
chacun des capteurs C1, C2 et C3 émet un signal en l'absence de ce ou ces analytes,
et l'adsorption dudit ou desdits analytes par le matériau sensible d'un capteur produisant une variation du signal émis par ce capteur ;
- des moyens d'acquisition et d'analyse pour acquérir et analyser, de manière synchrone et répétitive, des signaux émis par C1, C2 et C3 pendant une mise en contact du détecteur avec le milieu gazeux, afin d'obtenir, pour chaque capteur, une courbe représentant le signal émis par ce capteur en fonction du temps, cette courbe représentant soit la ligne de base du capteur dans le cas où le matériau sensible de ce capteur n'adsorbe aucun des analytes, soit une cinétique d'adsorption qui est caractéristique du nombre d'analyte(s) adsorbé(s) par le matériau sensible du capteur et de ce(s) analyte(s) ;
- des moyens de détection pour détecter une variation du signal émis par l'un au moins des capteurs C1, C2 et C3 au-delà d'au moins un seuil S, en valeur absolue, préalablement fixé pour le capteur émettant ce signal ;
- des moyens de comparaison pour comparer la cinétique d'adsorption obtenue pour le capteur ayant une variation du signal émis, en valeur absolue, supérieure au seuil S, avec une pluralité de modèles de cinétiques d'adsorption préalablement établis pour chacun des analytes susceptibles d'être détectés par ce capteur.

Les analytes à détecter et à analyser sont des molécules gazeuses sous forme de traces. Il peut par exemple s'agir d'explosifs, de composés entrant dans la fabrication d'explosifs ou signant la présence d'explosifs tels que les dérivés nitrés (par exemple, le nitrométhane), les composés nitroaromatiques (par exemple, le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène ou encore le trinitrophénol), les nitramines (par exemple, la cyclotétraméthylènetétranitramine (ou octogène), la cyclotriméthylènetrinitramine (ou hexogène) ou encore la trinitrophénylméthylnitramine (ou tétryle), les nitrasimes (par exemple, la nitrosodiméthylamine), les esters nitriques (par exemple, la pentrite, le dinitrate d'éthylène glycol (EGDN), le dinitrate de diéthylène glycol, la nitroglycérine ou de nitroguanidine) et les peroxydes (l'hexaméthylènetriperoxydediamine (HMTD), le triperoxyde de triacétone (TATP), le peroxyde d'hydrogène, etc.

Il peut également s'agir de polluants.

Le dispositif selon l'invention peut ainsi être utilisé pour la détection de composés autres que ceux liés aux aspects de la menace et la sécurité des lieux publics, comme la protection de l'environnement, en particulier pour le contrôle et la surveillance de la pollution atmosphérique et de la qualité d'ambiances, ainsi que pour, la surveillance à des fins sécuritaires de sites industriel.

L'invention sera mieux comprise et d'autres avantages et particularités apparaîtront à la lecture des exemples qui suivent et qui se réfèrent aux figures annexées.

Bien entendu, ces exemples ne sont donnés qu'à titre d'illustrations de l'objet de l'invention et ne constituent en aucun cas une limitation de cet objet.

**BRÈVE DESCRIPTION DES DESSINS**

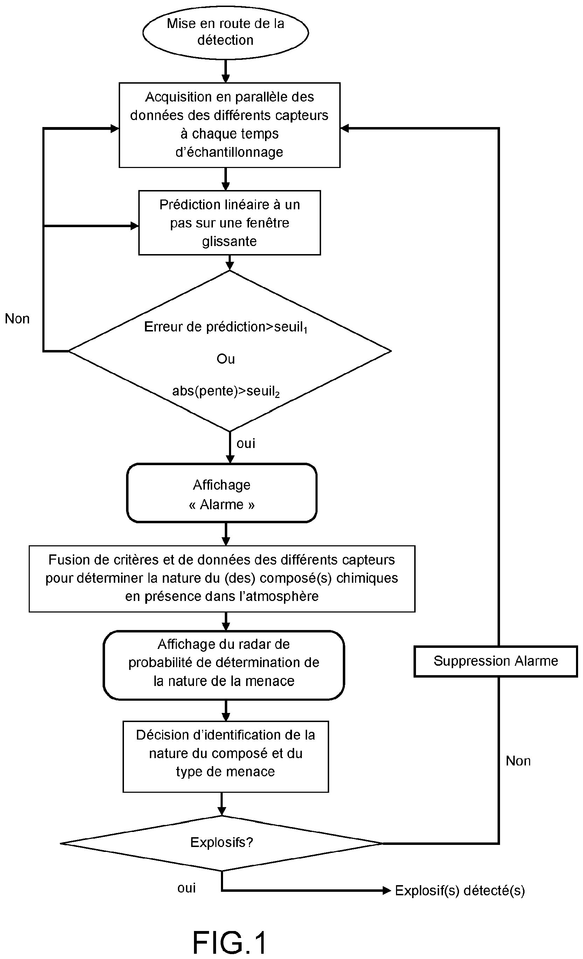

La figure 1 représente un exemple d'organigramme d'algorithme de détection selon l'invention permettant, dans un premier temps, d'évaluer la présence d'une menace, puis, dans un second temps, de déterminer sa nature chimique.

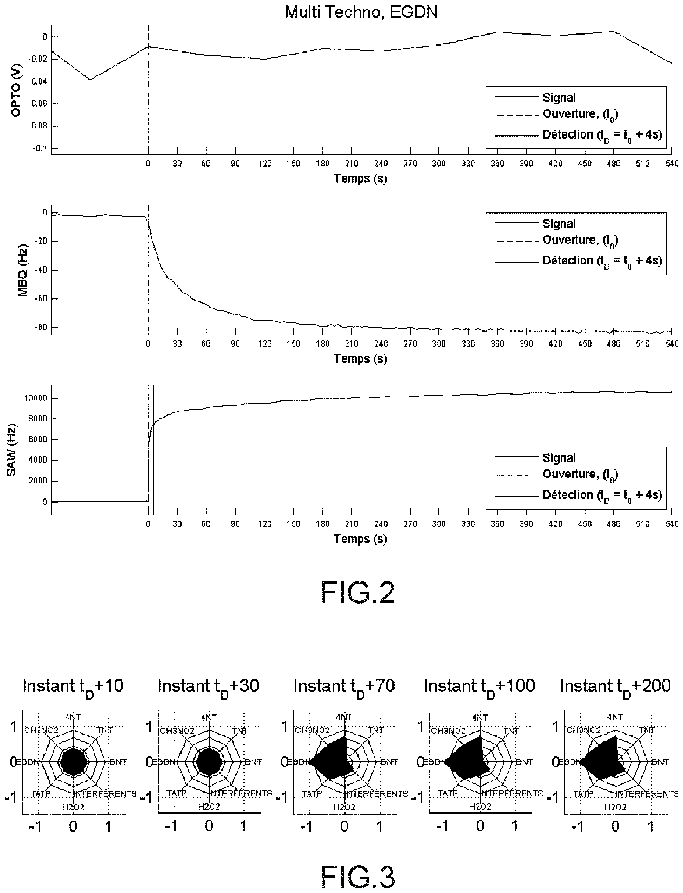

La figure 2 représente les signaux enregistrés respectivement par un capteur fluorescent, un capteur MBQ et un capteur SAW d'un dispositif de détection utilisé dans le procédé selon l'invention, suite à une exposition du dispositif à de l'air sec, puis à des vapeurs de dinitrate d'éthylène glycol (EGDN) dans de l'air sec, à 20°C et à une concentration de 100 ppm.

La figure 3 illustre l'évolution de l'étape d'identification de la nature chimique du composé présent dans le milieu gazeux à analyser à partir de l'instant de la détection avec le dispositif à trois transducteurs selon l'invention pour une exposition à des vapeurs d'EGDN dans de l'air sec, à 20°C et à une concentration de 100 ppm. Plus particulièrement, la figure 3 représente les évolutions du radar de probabilité, à différents temps ($t_D$+10, $t_D$+30, $t_D$+70, $t_D$+100, $t_D$+200, $t_D$ étant l'instant de détection et le temps indiqué étant en secondes), obtenues sur la base des erreurs de reconstruction relatives à l'exposition des capteurs fluorescent, MBQ et SAW du dispositif de détection selon l'invention à de l'air sec, puis à des vapeurs d'EGDN, ces radars permettant la détermination de la nature chimique de la menace en présence.

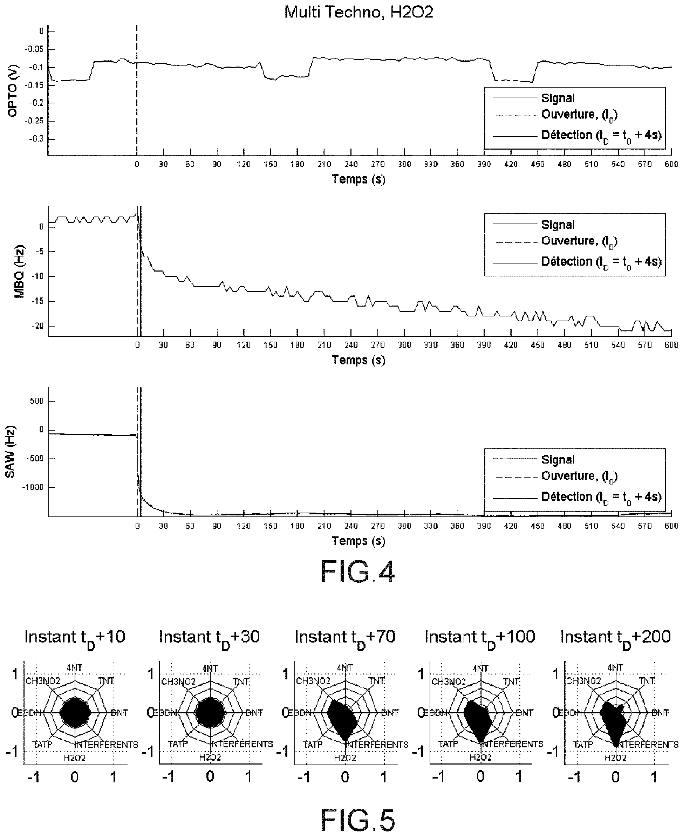

La figure 4 représente les signaux enregistrés respectivement par un capteur fluorescent, un capteur MBQ et un capteur SAW d'un dispositif de détection utilisé dans le procédé selon l'invention, suite à une exposition du dispositif à de l'air sec, puis à des vapeurs de peroxyde d'hydrogène ($H_2O_2$) dans de l'air sec, à 20°C et à une concentration de 500 ppm.

La figure 5 illustre l'évolution de l'étape d'identification de la nature chimique du composé présent dans le milieu gazeux à analyser à partir de l'instant de la détection avec le dispositif à trois transducteurs selon l'invention pour une exposition à des vapeurs d'$H_2O_2$ dans de l'air sec, à 20°C et à une concentration de 500 ppm. Plus particulièrement, la figure 5 représente les évolutions du radar de probabilité, à différents temps, qui permettent la détermination de la nature chimique de la menace en présence et qui sont obtenues sur la base des erreurs de reconstruction relative à l'exposition des capteurs fluorescent, MBQ et SAW du dispositif de détection selon l'invention à de l'air, puis à des vapeurs d'$H_2O_2$.

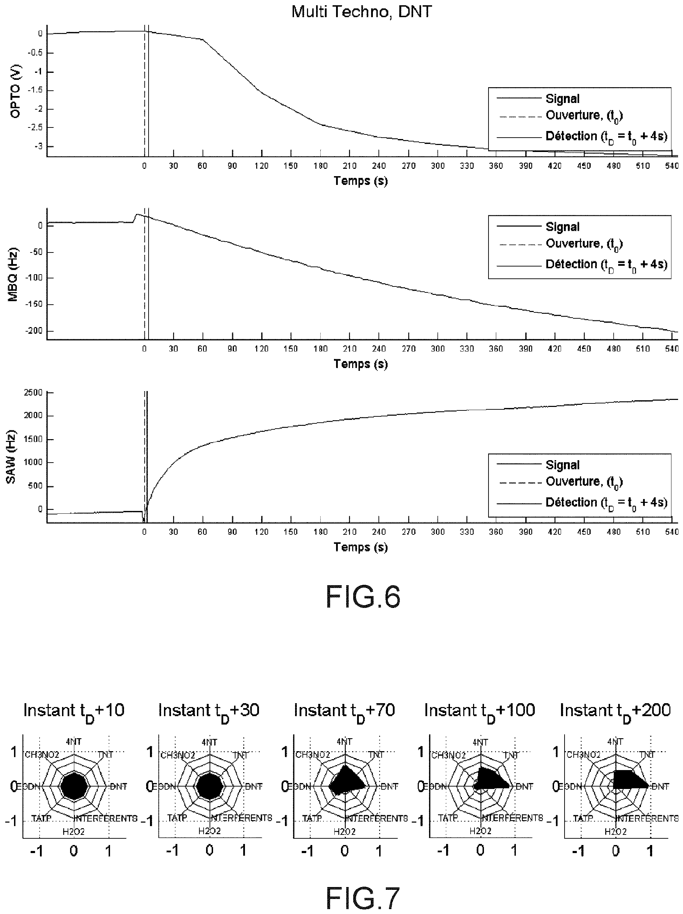

La figure 6 représente les signaux enregistrés respectivement par un capteur fluorescent, un capteur MBQ et un capteur SAW d'un dispositif de détection utilisé dans le procédé selon l'invention, suite à une exposition du dispositif selon l'invention à de l'air sec, puis à des vapeurs de dinitrotoluène (2,4-DNT) dans de l'air sec, à 20°C et à une concentration de 300 ppb.

La figure 7 illustre l'évolution de l'étape d'identification de la nature chimique du composé présent dans le milieu gazeux à analyser à partir de l'instant de la détection avec le dispositif à trois transducteurs selon l'invention pour une exposition à des vapeurs de 2,4-DNT dans de l'air sec, à 20°C et à une concentration de 300 ppb. Plus particulièrement, la figure 7 représente les évolutions du radar de probabilité permettant la détermination de la nature chimique de la menace en présence à différents temps et qui sont obtenues sur la base des erreurs de reconstruction relative à l'exposition des capteurs fluorescent, MBQ et SAW du dispositif de détection selon l'invention à de l'air sec, puis à des vapeurs de 2,4-DNT.

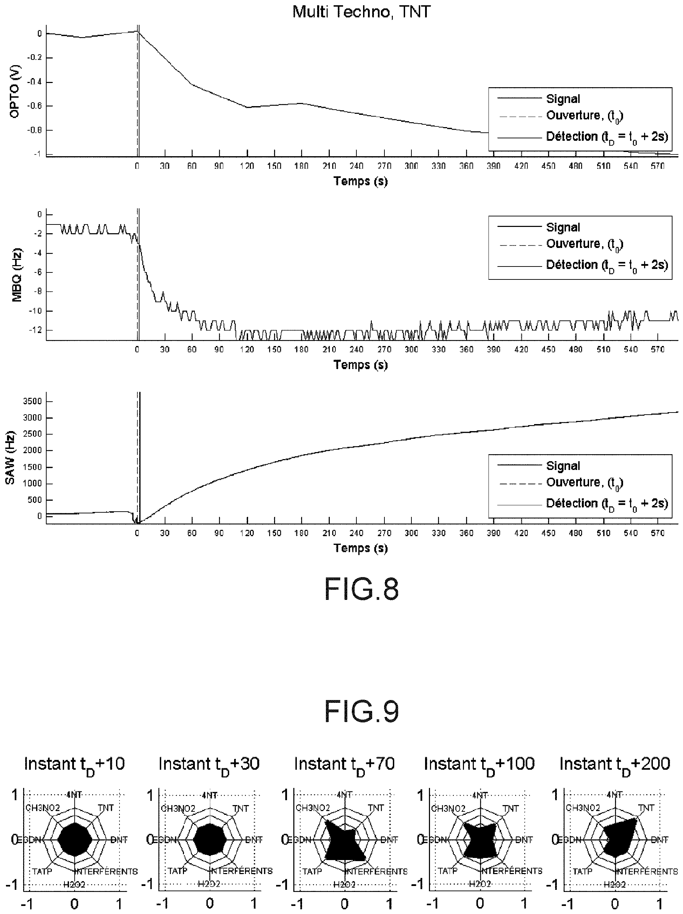

La figure 8 représente les signaux enregistrés respectivement par un capteur fluorescent, un capteur MBQ et un capteur SAW d'un dispositif de détection utilisé dans le procédé selon l'invention, suite à une exposition du dispositif à de l'air sec, puis à des vapeurs de trinitrotoluène (TNT) dans de l'air sec, à 20°C et à une concentration de 3 ppb.

La figure 9 illustre l'évolution de l'étape d'identification de la nature chimique du composé présent dans le milieu gazeux à analyser à partir de l'instant de la détection avec le dispositif à trois transducteurs selon l'invention pour une exposition à des vapeurs de TNT dans de l'air sec, à 20°C et à une concentration de 3 ppb. Plus particulièrement, la figure 9 représente les évolutions du radar de probabilité, à différents temps, qui permettent la détermination de la nature chimique de la menace en présence à différents temps et qui sont obtenues sur la base des erreurs de reconstruction relative à l'exposition des capteurs fluorescent, MBQ et SAW du dispositif de détection selon l'invention à de l'air sec, puis à des vapeurs de TNT.

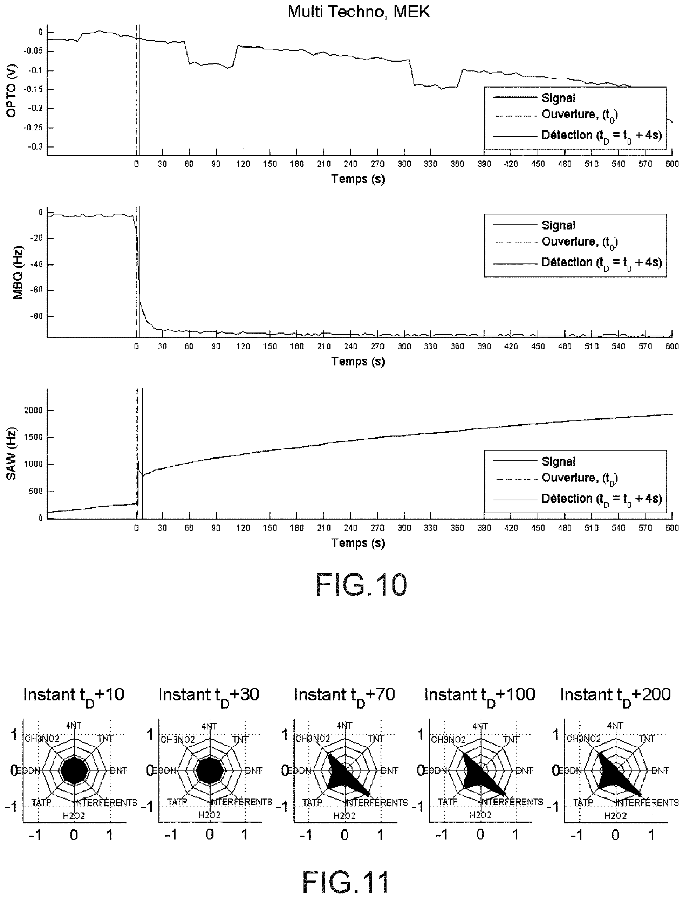

La figure 10 représente les signaux enregistrés respectivement par un capteur fluorescent, un capteur MBQ et un capteur SAW d'un dispositif de détection utilisé dans le procédé selon l'invention, suite à une exposition du dispositif à de l'air sec, puis à des vapeurs d'un interfèrent, la méthyléthylcétone (MEK), dans de l'air sec, à 20°C et à une concentration de 120 000 ppm.

La figure 11 illustre l'évolution de l'étape d'identification de la nature chimique du composé présent dans le milieu gazeux à analyser à partir de l'instant de la détection avec le dispositif à trois transducteurs selon l'invention pour une exposition à des vapeurs de MEK dans de l'air sec, à 20°C et à une concentration de 120 000 ppm. Plus particulièrement, la figure 11 représente les évolutions du radar de probabilité, à différents temps, qui permettent la détermination de la nature chimique de la menace en présence à différents temps et qui sont obtenues sur la base des erreurs de reconstruction relative à l'exposition des capteurs fluorescent, MBQ et SAW du dispositif de détection selon l'invention à de l'air sec, puis à des vapeurs de MEK.

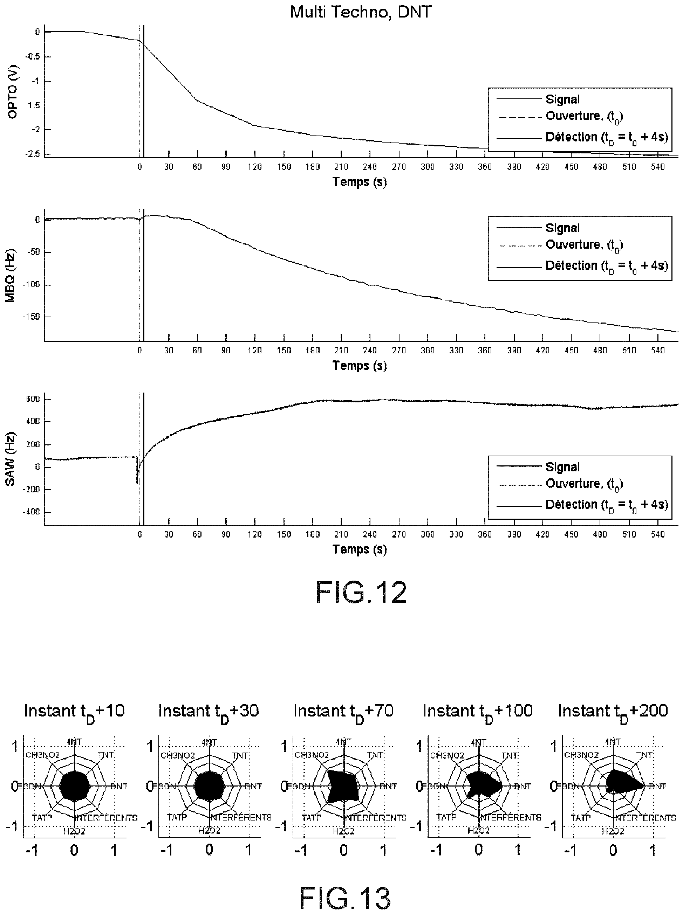

La figure 12 représente les signaux enregistrés respectivement par un capteur fluorescent, un capteur MBQ et un capteur SAW d'un dispositif de détection utilisé dans le procédé selon l'invention, suite à une exposition du dispositif à de l'air humide (50%), puis à des vapeurs de 2,4-DNT dans de l'air humide, à 20°C et à une concentration de 120 ppb.

La figure 13 illustre l'évolution de l'étape d'identification de la nature chimique du composé présent dans le milieu gazeux à analyser à partir de l'instant de la détection avec le dispositif à trois transducteurs selon l'invention pour une exposition à des vapeurs de 2,4-DNT dans de l'air humide (50%), à 20°C et à une concentration de 120 ppb. Plus particulièrement, la figure 13 représente les évolutions du radar de probabilité, à différents temps, qui permettent la détermination de la nature chimique de la menace en présence et qui sont obtenues sur la base des erreurs de reconstruction relative à l'exposition des capteurs fluorescent, MBQ et SAW du dispositif de détection selon l'invention à de l'air humide, puis à des vapeurs de 2,4-DNT en présence d'humidité.

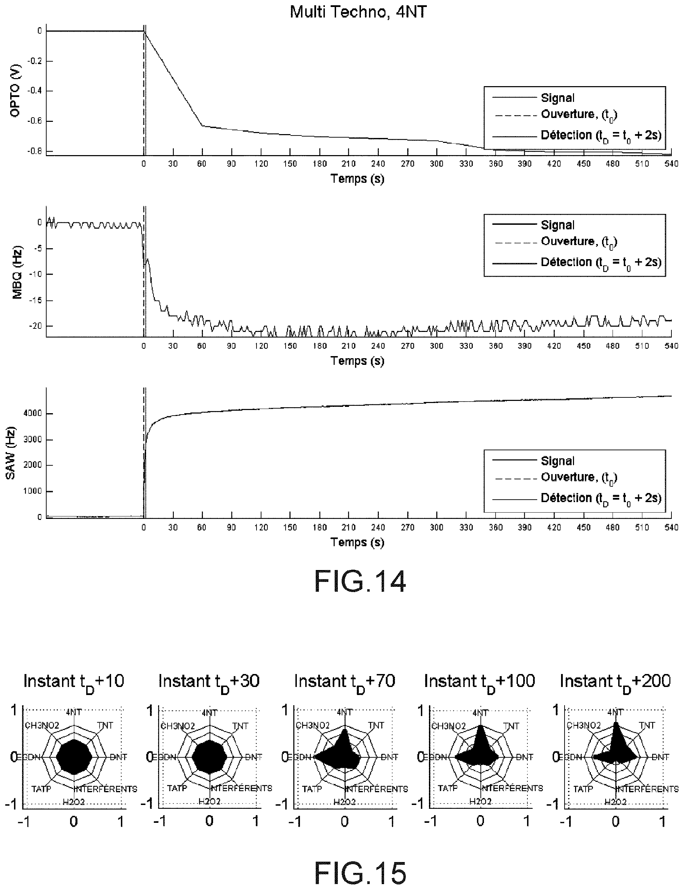

La figure 14 représente les signaux enregistrés respectivement par un capteur fluorescent, un capteur MBQ et un capteur SAW d'un dispositif de détection utilisé dans le procédé selon l'invention, suite à une exposition du dispositif à de l'air sec, puis à des vapeurs de nitrotoluène (4-NT) dans de l'air sec, à 20°C et à une concentration de 79 ppm.

La figure 15 illustre l'évolution de l'étape d'identification de la nature chimique du composé présent dans le milieu gazeux à analyser à partir de l'instant de la détection avec le dispositif à trois transducteurs selon l'invention pour une exposition à du 4-NT (dans de l'air sec, à 20°C et à une concentration de 79 ppm. Plus particulièrement, la figure 15 représente les évolutions du radar de probabilité, à différents temps, qui permettent la détermination de la nature chimique de la menace en présence et qui sont obtenues sur la base des erreurs de reconstruction relative à l'exposition des capteurs fluorescent, MBQ et SAW du dispositif de détection selon l'invention à de l'air sec, puis à des vapeurs de nitrotoluène.

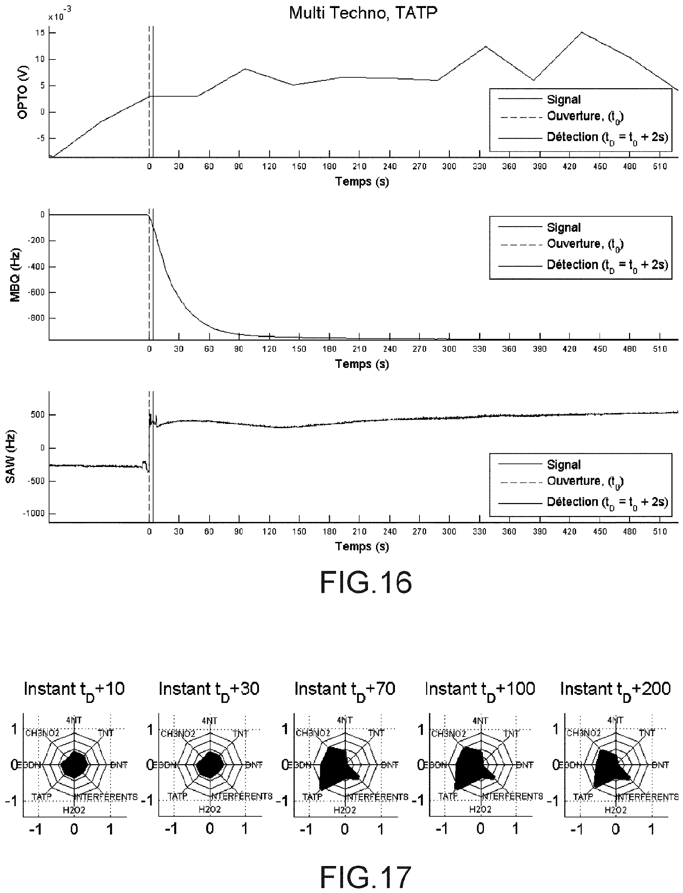

La figure 16 représente les signaux enregistrés respectivement par un capteur fluorescent, un capteur MBQ et un capteur SAW d'un dispositif de détection utilisé dans le procédé selon l'invention, suite à une exposition du dispositif à de l'air humide (40-50%), puis à des vapeurs de tripéroxyde de triacétone (TATP) dans de l'air humide (40-50%), à 20°C et à une concentration de 50 ppm.

La figure 17 illustre l'évolution de l'étape d'identification de la nature chimique du composé présent dans le milieu gazeux à analyser à partir de l'instant de la détection avec le dispositif à trois transducteurs selon l'invention pour une exposition à du TATP en présence d'humidité. Plus particulièrement, la figure 17 représente les évolutions du radar de probabilité, à différents temps, qui permettent la détermination de la nature chimique de la menace en présence et qui sont obtenues sur la base des erreurs de reconstruction relative à l'exposition des capteurs fluorescent, MBQ et SAW du dispositif de détection selon l'invention à de l'air humide (50%), puis à des vapeurs de TATP dans de l'air humide (50%), à 20°C et à une concentration de 50 ppm.

Il est à noter que dans les figures 3, 5, 7, 9, 11, 13, 15 et 17, les signaux enregistrés représentent respectivement l'évolution de l'intensité de fluorescence du capteur fluorescent, de fréquence de résonance du capteur MBQ et de fréquence de résonance du capteur SAW, au cours du temps, en fonction de la composition du milieu gazeux. Dans ces figures, $t_0$ correspond à l'instant de mise en contact du milieu gazeux à analyser avec les trois capteurs du détecteur utilisé et $t_D$ est l'instant de détection. L'instant $t_0$ peut par exemple correspondre à l'instant où l'on procède à l'ouverture d'une vanne permettant l'arrivée du milieu gazeux à analyser dans la cellule de mesure contenant les trois capteurs.

## EXPOSÉ DÉTAILLÉ DE MODES DE RÉALISATION PARTICULIERS

Comme nous l'avons vu ci-dessus, le procédé de détection et d'identification selon l'invention nécessite l'utilisation d'un détecteur comprenant au moins trois capteurs chimiques différents, formés respectivement d'un transducteur optique, d'un premier transducteur gravimétrique et d'un deuxième transducteur gravimétrique. De préférence, les trois transducteurs sont un transducteur de fluorescence, un transducteur MBQ (microbalance à quartz) et un transducteur SAW (à ondes acoustiques).

Il est bien entendu que le choix du matériau sensible de la couche de chaque capteur est fait en fonction des molécules cibles que l'on souhaite détecter.

Afin de démontrer l'intérêt du procédé selon l'invention, nous allons à présent appliquer ledit procédé pour réaliser la détection et l'identification de diverses molécules sous forme gazeuse, le détecteur utilisé dans les exemples ci-dessous comprenant uniquement trois capteurs différents, à savoir un capteur MBQ, un capteur fluorescent et un capteur SAW, placés en parallèle dans une seule et même cellule de mesure.

Le capteur MBQ est ici réalisé en recouvrant les deux faces d'un substrat en quartz de coupe AT, de fréquence de résonance 9 MHz, muni de deux électrodes circulaires de mesure en or (par exemple, la référence QA9RA-50, de chez AMETEK PRECISION INSTRUMENTS), d'un film mince de silice fonctionnalisée par une méthylation de surface.

Le dépôt du film mince peut être réalisé par trempage-retrait d'une solution de silice fonctionnalisée dans l'éthanol (concentration à 3% en masse) sur les deux faces de la microbalance à quartz. La diminution de fréquence de vibration de la microbalance à quartz due à ce dépôt est de 10 kHz.

Le capteur fluorescent est, quant à lui, réalisé en recouvrant une face d'une lame de verre d'un film mince de diimine phénylèneéthynylène tel que décrit dans le document **[8]** par enduction centrifuge d'une solution de ce composé dans du tétrahydrofurane (THF) de concentration à 2,5 g/L, à une vitesse de rotation de 600 tours par minute. L'épaisseur de la couche est de l'ordre de 50 nm. Le film ainsi obtenu présente une fluorescence à une longueur d'onde de 480 nm lorsqu'il est excité par une lumière émettant dans l'UV (typiquement à 380 nm).

Le capteur SAW est réalisé en recouvrant un substrat piézoélectrique de fréquence propre 433 MHz (de marque Forschungszentrum Karlsruhe IMT/SAGAS), d'un film mince de $PcZn(Oct)_8$ (c'est-à-dire de l'octo(octyoxy)phtalocyanine de Zinc, n°CAS 261504-18-1). Le dépôt de ce film est réalisé par pulvérisation d'une solution de $PcZn(Oct)_8$ dans du dichlorométhane à 1,5 g/L. La diminution de fréquence de vibration du substrat piézoélectrique due à ce dépôt est de 100 kHz.

**Exemple 1** : **exposition du dispositif à de l'air sec, puis à des vapeurs d'EGDN dans de l'air sec**

Les trois capteurs du dispositif de détection sont exposés simultanément à de l'air synthétique sec pendant 5 minutes, puis à des vapeurs d'EGDN, à une concentration de 100 ppm dans de l'air synthétique sec, pendant 9 minutes. On rappelle que de l'air synthétique sec est un air contenant 0% d'humidité.

Ces expositions sont réalisées à 20°C et en utilisant des débits gazeux de 20 L/h.

Dans cet exemple, la détection est réalisée 4 secondes après la mise en contact des trois capteurs avec l'atmosphère à analyser ($t_D = t_0 + 4s$).

Dans la figure 2, on constate que l'exposition à l'EGDN entraîne une variation des fréquences propres des capteurs MBQ et SAW (respectivement de 80 Hz et de 9 kHz, après 9 minutes d'exposition à l'atmosphère contenant de l'EGDN), alors que l'intensité de fluorescence du capteur fluorescent ne varie pratiquement pas.

A partir de ces courbes, on peut déterminer la cinétique de chaque capteur vis-à-vis de la vapeur à laquelle il est exposé.

Dans tous les cas, la réponse R du capteur peut être modélisée par la relation :

$$R(t=temps) = A\ (exp(-t/tau)-1)\ +\ B$$

dans laquelle A et B sont des constantes positives ou négatives selon le type de capteur.

La cinétique est, en secondes, la valeur que prend tau. Si tau est faible, la cinétique est particulièrement rapide (cas général du SAW) ; dans le cas contraire, elle est lente (cas du capteur fluorescent).

On constate également que les cinétiques d'adsorption de l'EGDN sont différentes entre les capteurs SAW et MBQ (respectivement de l'ordre de 1 seconde et de 30 secondes).

Généralement, un signal est considéré comme significatif si :

- il est supérieur, en valeur absolue, à 10 Hz pour 9 minutes en QCM ;
- il est supérieur, en valeur absolue, à 100 Hz pour 9 minutes en SAW ;
- il est supérieur, en valeur absolue, à 0.2 V en 9 minutes en fluorescence.

Les évolutions du radar de probabilité nous permettent de déterminer la nature chimique du composé introduit dans la cellule de mesure du dispositif de détection.

Les composés présents dans le radar sont les cibles du type explosifs que l'on cherche à détecter, par exemple :

- EGDN

- TNT

- 4-NT

- DNT

- $H_2O_2$

- TATP

- $CH_3NO_2$

ainsi que des interférents qui sont, par exemple, les solvants présents dans les parfums.

Compte tenu des erreurs de reconstruction, le dispositif se met à un premier niveau d'alarme, informant l'utilisateur d'une menace potentielle, en 4 secondes (figure 2) ; puis, grâce aux évolutions du radar de probabilité, on détermine, en 70 secondes après la détection (instant $t_D$+70 ; figure 3), que le composé présent dans l'atmosphère à analyser est du EDGN.

Il est à noter qu'au-delà de 70 secondes après la détection, nous ne notons plus d'évolution de la vue radar du vecteur de probabilité (figure 3).

En combinant les trois capteurs proposés dans le présent exemple, associés aux matériaux sensibles adaptés, et en comparant leurs cinétiques d'adsorption de l'EGDN, il est possible d'évaluer rapidement la présence de la menace (10 à 20 secondes après l'introduction de l'échantillon dans la cellule de mesure du dispositif) et de déterminer la nature du composé chimique présent dans l'atmosphère de l'échantillon introduit (en 1 à 2 minutes (ici en 70 secondes)). Le procédé selon l'invention permet donc bien de déterminer, rapidement et avec une bonne fiabilité, la nature chimique du composé présent dans l'atmosphère.

### Exemple 2 : exposition du dispositif à de l'air sec, puis à des vapeurs de peroxyde d'hydrogène ($H_2O_2$) dans de l'air sec

Les trois capteurs du dispositif sont exposés simultanément à de l'air synthétique sec (0% d'humidité) pendant 5 minutes, puis à des vapeurs d'$H_2O_2$, à une concentration de 500 ppm dans de l'air synthétique sec, pendant 10 minutes.

Ces expositions sont réalisées à 20°C et en utilisant des débits gazeux de 20 L/h.

En étudiant les courbes de la figure 4, on constate que l'exposition à l'$H_2O_2$ entraîne une variation des fréquences propres des capteurs MBQ et SAW (respectivement de 20 Hz et de 1500 Hz en négatif après 10 minutes d'exposition), alors que l'intensité de fluorescence du capteur fluorescent ne varie pratiquement pas.

En outre, on constate que les cinétiques d'adsorption de l'$H_2O_2$ sont différentes entre les capteurs SAW et MBQ (de l'ordre de 1 seconde pour le SAW et 25 secondes pour le MBQ).

Compte tenu des erreurs de reconstruction, le dispositif se met à un premier niveau d'alarme, informant l'utilisateur d'une menace potentielle, en 4 secondes, puis permet de déterminer, avec une bonne fiabilité, la nature chimique du composé présent dans l'atmosphère en 70 secondes après la détection (figure 5) .

Ainsi, en combinant les trois capteurs proposés dans le présent exemple, associés aux matériaux sensibles adaptés, et en comparant leurs cinétiques d'adsorption de l'$H_2O_2$, il est possible d'évaluer rapidement la présence de la menace (10 à 20 secondes après l'exposition du dispositif à l'atmposphère à analyser) et de déterminer rapidement (1 à 2 minutes après le début de la détection) la nature du composé chimique présent dans l'atmosphère.

### Exemple 3 : exposition du dispositif à de l'air sec, puis à des vapeurs de dinitrotoluène (2,4-DNT) dans de l'air sec

Les trois capteurs sont exposés simultanément à de l'air synthétique sec (0% d'humidité) pendant 5 minutes, puis à des vapeurs de 2,4-DNT, à une concentration de 300 ppb dans de l'air synthétique sec, pendant 10 minutes.

Ces expositions sont réalisées à 20°C et en utilisant des débits gazeux de 20 L/heure.

En étudiant les courbes de la figure 6, on constate que l'exposition au 2,4-DNT entraîne une variation des fréquences propres des capteurs MBQ et SAW (respectivement de 200 Hz et de 2000 Hz en négatif après 9 minutes d'exposition) et de l'intensité de fluorescence du capteur fluorescent (correspondant à 3 V) .

En outre, on constate que les cinétiques d'adsorption du 2,4-DNT sont différentes entre les capteurs fluorescent, MBQ et SAW (1000 secondes pour le capteur fluorescent, 250 secondes pour le MBQ et 30 secondes pour le SAW).

Compte tenu des erreurs de reconstruction, le dispositif se met à un premier niveau d'alarme, informant l'utilisateur d'une menace potentielle, en 4 secondes, puis permet de déterminer, avec une bonne fiabilité, la nature chimique du composé présent dans l'atmosphère en 100 secondes (figure 7).

Ainsi, en combinant les trois capteurs proposés dans le présent exemple, associés aux matériaux sensibles adaptés, et en comparant leurs cinétiques d'adsorption du 2,4-DNT, il est possible d'évaluer rapidement la présence de la menace (10 à 20 secondes après l'exposition du dispositif à l'atmposphère à analyser) et de déterminer rapidement (1 à 2 minutes après le début de la détection) la nature du composé chimique présent dans l'atmosphère.

### Exemple 4 : exposition du dispositif à de l'air sec, puis à des vapeurs de trinitrotoluène (TNT) dans de l'air sec

Les trois capteurs sont exposés simultanément à de l'air synthétique sec (0% d'humidité) pendant 5 minutes, puis à des vapeurs de TNT, à une concentration de 3 ppb dans de l'air synthétique sec, pendant 10 minutes.

Ces expositions sont réalisées à 20°C et en utilisant des débits gazeux de 20 L/heure.

En étudiant les courbes de la figure 8, on constate que l'exposition au TNT entraîne une variation de l'intensité de fluorescence du capteur fluorescent (correspondant à 0,8 V) et une variation de la fréquence propre du capteur SAW (de 3000 Hz après 10 minutes d'exposition). La variation du signal du capteur MBQ est, quant à elle, très faible (seulement 12 Hz d'amplitude après 10 minutes d'exposition). Cette faible amplitude de signal peut être confondue avec une non-adsorption de la part du capteur.

En outre, on constate que les cinétiques d'adsorption du TNT sont différentes entre les capteurs fluorescent et SAW. Le capteur SAW a une cinétique de 110 secondes, tandis que le capteur fluorescent a une cinétique de 100 000 secondes. Compte-tenu de la cinétique relativement longue du capteur fluorescent, on réalisera qu'une seule détection.

Compte tenu des erreurs de reconstruction, le dispositif se met à un premier niveau d'alarme, informant l'utilisateur d'une menace potentielle, en 2 secondes, puis permet de déterminer, avec une bonne fiabilité, la nature chimique du composé présent dans l'atmosphère en 200 secondes (figure 9).

On notera que l'identification est plus longue dans cet exemple, car les cinétiques des signaux sont très longues et l'intensité des signaux est faible en raison de la faible pression de vapeur du TNT.

En combinant les trois capteurs proposés dans le présent exemple, associés aux matériaux sensibles adaptés, et en comparant leurs cinétiques d'adsorption du TNT, il est possible d'évaluer rapidement la présence de la menace (10 à 20 secondes après l'exposition du dispositif à l'atmosphère à analyser) et de déterminer la nature du composé chimique présent dans l'atmosphère en moins de 3 minutes après le début de la détection.

**Exemple 5 : exposition du dispositif à de l'air sec, puis à des vapeurs d'un interférent (méthyléthylcétone) dans de l'air sec**

Les trois capteurs sont exposés simultanément à de l'air synthétique sec (0% d'humidité) pendant 5 minutes, puis à des vapeurs de méthyléthylcétone, à une concentration de 120 000 ppm dans de l'air synthétique sec, pendant 10 minutes.

Ces expositions sont réalisées à 20°C et en utilisant des débits gazeux de 20 L/heure.

En étudiant les courbes de la figure 10, on constate que l'exposition à la méthyléthylcétone entraîne une variation des fréquences propres des capteurs MBQ et SAW (respectivement 80 Hz et 1500 Hz après 10 minutes d'exposition), alors que l'intensité de fluorescence du capteur fluorescent ne varie pratiquement pas.

En outre, on constate que les cinétiques d'adsorption de la méthyléthylcétone sont différentes entre les capteurs MBQ et SAW (environ 5 secondes pour le MBQ et 1 seconde pour le SAW).

Compte tenu des erreurs de reconstruction, le dispositif se met à un premier niveau d'alarme, informant l'utilisateur d'une menace potentielle, en 4 secondes, puis permet de déterminer, avec une bonne fiabilité, qu'il s'agit d'un interférent présent dans l'atmosphère en 70 secondes (figure 11) et que l'alerte peut être arrêtée.

Il est à noter que si nous pouvions réduire au minimum la sensibilité des capteurs à ce type de composés interférents, alors l'alerte ne serait même pas générée.

Il existe d'autres matériaux sensibles, autre que la silice utilisée dans cet exemple, qui permettent de palier à ce problème. Cependant, ils n'ont pas une aussi bonne sensibilité aux explosifs ou aux précurseurs que la silice.

Au final, en combinant les trois capteurs proposés dans le présent exemple, associés aux matériaux sensibles adaptés, et en comparant leurs cinétiques d'adsorption de la méthyléthylcétone, il est possible d'évaluer rapidement la présence de la menace potentielle (10 à 20 secondes après l'exposition du dispositif à l'atmosphère à analyser), pour déterminer rapidement (1 à 2 minutes après le début de la détection) que cette menace est en fait un interférent présent dans l'atmosphère.

**Exemple 6 : exposition du dispositif à de l'air humide, puis à des vapeurs de dinitrotoluène (2,4-DNT) en présence d'humidité**

Les trois capteurs sont exposés simultanément à de l'air synthétique humide (50% d'humidité) pendant 5 minutes, puis à des vapeurs humides (50% d'humidité) de 2,4-DNT, à une concentration de 120 ppb, pendant 9 minutes.

Ces expositions sont réalisées à 20°C et en utilisant des débits gazeux de 20 L/heure.

En étudiant les courbes de la figure 12, on constate que l'exposition au 2,4-DNT entraîne une variation de l'intensité de fluorescence du capteur fluorescent (correspondant à 2,5 V) et une variation des fréquences propres des capteurs MBQ et SAW (respectivement de 150 Hz et 600 Hz après 9 minutes d'exposition).

En outre, on constate que les cinétiques d'adsorption du 2,4-DNT sont différentes entre les capteurs fluorescent, MBQ et SAW. Comme les cinétiques d'aborption sont indépendantes de l'humidité, elles sont les mêmes que celles précisées dans l'exemple 3.

Compte tenu des erreurs de reconstruction, le dispositif se met à un premier niveau d'alarme, informant l'utilisateur d'une menace potentielle, en 4 secondes, puis permet de déterminer, avec une bonne fiabilité, la nature chimique du composé présent dans l'atmosphère en 200 secondes (figure 13).

On notera que l'identification est plus lente que dans l'exemple 3 sous air sec, car les signaux ont une amplitude plus petite en raison de l'humidité.

En combinant les trois capteurs proposés dans le présent exemple, associés aux matériaux sensibles adaptés, et en comparant leurs cinétiques d'adsorption du 2,4-DNT, il est possible d'évaluer rapidement la présence de la menace (10 à 20 secondes après l'exposition du dispositif à l'atmosphère à analyser) et de déterminer rapidement (en moins de 3 minutes après le début de la détection) la nature du composé chimique présent dans l'atmosphère, même en présence d'humidité dans l'atmosphère à analyser.

**Exemple 7 : exposition du dispositif à de l'air sec, puis à des vapeurs de nitrotoluène (4-NT) dans de l'air sec**

Les trois capteurs sont exposés simultanément à de l'air synthétique sec (0% d'humidité) pendant 5 minutes, puis à des vapeurs de 4-NT, à une concentration de 79 ppm dans de l'air synthétique sec, pendant 9 minutes.

Ces expositions sont réalisées à 20°C et en utilisant des débits gazeux de 20 L/heure.

En étudiant les courbes de la figure 14, on constate que l'exposition au 4-NT entraîne une variation de la fréquence propre du capteur SAW (4000 Hz après 9 minutes d'exposition) et de faibles variation des signaux des capteurs MBQ et fluorescent (de l'ordre de 20 Hz et de 0,8 V, respectivement).

En outre, on constate que les cinétiques d'adsorption du 4-NT sont différentes entre les capteurs fluorescent (de l'ordre de 700 secondes), MBQ et SAW (de l'ordre de 2 secondes).

Compte tenu des erreurs de reconstruction, le dispositif se met à un premier niveau d'alarme, informant l'utilisateur d'une menace potentielle, en 2 secondes, puis permet de déterminer, avec une bonne fiabilité, qu'il s'agit d'un marqueur présent dans l'atmosphère en 200 secondes (figure 15).

En combinant les trois capteurs proposés dans le présent exemple, associés aux matériaux sensibles adaptés, et en comparant leurs cinétiques d'adsorption du 4-NT, il est possible d'évaluer rapidement la présence de la menace potentielle (10 à 20 secondes après l'exposition du dispositif à l'atmosphère à analyser) et de déterminer rapidement (en moins de 3 minutes après le début de la détection) que cette menace potentielle correspond à un marqueur d'explosifs présent dans l'atmosphère.

**Exemple 8 : exposition du dispositif à de l'air humide, puis à des vapeurs de tripéroxyde de triacétone (TATP) en présence d'humidité**

Les trois capteurs sont exposés simultanément à de l'air synthétique humide (40% à 50% d'humidité à 20°C) pendant 5 minutes, puis à des vapeurs humides (40 % à 50% d'humidité à 20°C) de TATP, à une concentration de 50 ppm, pendant 9 minutes.

Ces expositions sont réalisées à 20°C et en utilisant des débits gazeux de 20 L/heure.

En étudiant les courbes de la figure 16, on constate que l'exposition au TATP entraîne une variation des fréquences propres des capteurs MBQ et SAW (respectivement de 900 Hz et 500 Hz après 9 minutes d'exposition), alors que le signal issu du capteur fluorescent n'évolue pas.

En outre, on constate que les cinétiques d'adsorption du TATP sont différentes entre les capteurs MBQ et SAW : elle est de 30 secondes pour le capteur MBQ et elle est plus rapide pour le capteur SAW (de l'ordre de la seconde).

Compte tenu des erreurs de reconstruction, le dispositif se met à un premier niveau d'alarme, informant l'utilisateur d'une menace potentielle, en 2 secondes, puis permet de déterminer, avec une bonne fiabilité, qu'il s'agit d'un interférent présent dans l'atmosphère en 70 secondes (figure 17).

En combinant les trois capteurs proposés dans le présent exemple, associés aux matériaux sensibles adaptés, et en comparant leurs cinétiques d'adsorption du TATP, il est possible d'évaluer rapidement la présence de la menace (10 à 20 secondes après l'exposition du dispositif à l'atmosphère à analyser) et de déterminer rapidement (1 à 2 minutes après le début de la détection) - même en présence d'humidité dans l'atmosphère à analyser - que cette menace est en fait un interférent présent dans l'atmosphère.

**Conclusion des exemples ci-dessus**

Les différents exemples cités ci-dessus démontrent qu'avec un seul dispositif de détection selon l'invention, comportant un capteur MBQ, un capteur fluorescent et un capteur SAW, associés aux matériaux sensibles mentionnés, et en procédant à une discrimination des cinétiques d'adsorption des molécules cibles sur le matériau sensible des différents capteurs, il est possible d'être alerté rapidement (typiquement en environ 10 secondes) de la présence potentielle d'une molécule cible néfaste (polluant, explosif, etc) et d'en déterminer la nature en moins de 3 minutes. Cela est particulièrement avantageux pour la détection d'explosifs, où il est important d'alerter rapidement les équipes de première intervention d'une menace d'explosion.

Le procédé selon l'invention permet en outre de discriminer et de déterminer, de manière fiable, la nature des différents composés, explosifs, précurseurs ou marqueurs présents dans l'atmosphère à analyser.

Il est à noter que, en plus du capteur optique et des deux capteurs gravimétriques différents, il est possible d'ajouter des capteurs de gaz supplémentaires dans la cellule de mesure du dispositif de détection utilisé dans le procédé selon l'invention, afin d'améliorer la fiabilité de la détection et/ou d'élargir la famille de molécules cibles qu'il est possible de détecter et d'identifier. Il peut s'agir de capteurs à haute sensibilité comme les capteurs fluorescents, MBQ et SAW, ou bien de capteurs traditionnels, comme les capteurs résistifs, électrochimiques, les capteurs à micro-levier, les capteurs semiconducteurs à base d'oxydes métalliques, etc.

## BIBLIOGRAPHIE

[1] US 2008/0085212 A1
*"Cantilevered probe detector with piezoelectric element"*

[2] D. S. Lee et al."Explosive gas recognition system using thick film sensor array and neural network", Sensors and Actuators B: Chemical, 71 (2000), p. 90

[3] US 6,636,811 B1
*"Method and device for identifying gaseous compounds"*

[4] J. Park et al."Vapor recognition with small arrays of polymer-coated microsensor. A comprehensive analysis", Anal. Chem., 71 (1999), p. 3877

[5] M. Burl et al."Classification performance of carbon black-polymer composite vapor detector arrays as a function of array size and detector composition", Sensors and Acuators B: Chemical, 87 (2002), p. 130

[6] WO 00/16096 A1
*"Artificial olfactory sensing system"*

[7] US 2006/0219892 A1
*"Method and arrangement for detecting harmful substances"*

[8] FR 2 868 842
*"Capteurs chimiques comprenant des polymères conjugués fluorescents comme matériaux sensibles, et leur utilisation pour la détection et le dosage de composés nitrés"*

## Revendications

1.   Procédé de détection et d'identification d'un analyte présent dans un milieu gazeux contenant un ou plusieurs analytes, dans lequel :

on utilise un détecteur qui comprend au moins trois capteurs C1, C2 et C3, comprenant chacun un transducteur associé à un matériau sensible capable d'adsorber un ou plusieurs analytes,
C1 comprend un transducteur optique tandis que C2 et C3 comprennent des transducteurs gravimétriques différents l'un de l'autre,
chacun des capteurs C1, C2 et C3 émet un signal en l'absence de ce ou ces analytes,
et l'adsorption dudit ou desdits analytes par le matériau sensible d'un capteur produisant une variation du signal émis par ce capteur ;
le procédé comprenant :

a) la mise en contact du détecteur avec le milieu gazeux ;

**caractérisé en ce que** le procédé comprend aussi:
b) l'acquisition et l'analyse, de manière synchrone et répétitive, des signaux émis par C1, C2 et C3 pendant cette mise en contact pour obtenir, pour chaque capteur, une courbe représentant le signal émis par ce capteur en fonction du temps, cette courbe représentant soit la ligne de base du capteur dans le cas où le matériau sensible de ce capteur n'adsorbe aucun des analytes, soit une cinétique d'adsorption qui est caractéristique du nombre d'analyte(s) adsorbé(s) par le matériau sensible du capteur et de ce(s) analyte(s) ;

moyennant quoi :

si, au cours de l'étape b), une variation du signal émis par l'un au moins de C1, C2 et C3 au-delà d'au moins un seuil S, en valeur absolue, préalablement fixé pour le capteur émettant ce signal est détectée, alors on poursuit les étapes a) et b) et on identifie le ou les analytes adsorbés par le matériau sensible de ce capteur par comparaison de la cinétique d'adsorption obtenue pour ce capteur avec une pluralité de modèles de cinétiques d'adsorption préalablement établies pour chacun des analytes susceptibles d'être détectés par ce capteur.

2.  Procédé de détection et d'identification selon la revendication 1, dans lequel la variation du signal détectée comprend une variation instantanée entre deux acquisitions du capteur successives.

3.  Procédé de détection et d'identification selon la revendication 1 ou la revendication 2, dans lequel la variation du signal détectée comprend une variation moyenne sur au moins trois acquisitions du capteur successives.

4.  Procédé de détection et d'identification selon la revendication 2 ou selon la revendication 3 prise en combinaison avec la revendication 2, dans lequel la détection d'une variation d'un signal au cours de l'étape b) comprend la réalisation, pour chaque capteur C1, C2 et C3, d'un calcul d'extrapolation linéaire d'une valeur extrapolée du $n^{\text{ième}}$ signal émis par ce capteur à partir des $(n-1)$ signaux précédemment émis par ce capteur, n étant un nombre entier supérieur ou égal à 3, la variation instantanée étant la différence en valeur absolue entre la valeur extrapolée et la vapeur acquise du $n^{\text{ième}}$ signal émis par ledit capteur, la détection du signal étant prise en compte lorsque la valeur, en valeur absolue, de la variation instantanée est supérieure ou égale à une valeur du seuil S1 préalablement fixé.

5.  Procédé de détection et d'identification selon la revendication 3 ou la revendication 4 prise en combinaison avec la revendication 3, dans lequel la détection d'une variation d'un signal au cours de l'étape b) comprend la réalisation, pour chaque capteur C1, C2 et C3, d'un calcul d'une pente à partir des $(n-1)$ signaux précédemment émis par le capteur, n étant un nombre entier supérieur ou égal à 3, la valeur absolue de cette pente correspondant à la variation moyenne, la détection étant prise en compte lorsque la valeur de la variation moyenne est supérieure à une valeur seuil S2 prédéterminée.

6.  Procédé de détection et d'identification selon la revendication 3, dans lequel l'identification est obtenue en réalisant les étapes successives suivantes :

    - pour chaque capteur et pour chaque modèle, calcul d'une courbe d'interpolation selon ledit modèle à partir des signaux dudit capteur acquis entre un temps $t_D$, correspondant au temps pour lequel une variation de signal émis par l'un au moins des capteurs C1, C2 et C3 est détectée, et un temps t ;
    - calcul d'erreurs pour chaque capteur et chaque modèle entre la courbe d'interpolation selon ledit modèle et les signaux dudit capteur acquis entre le temps $t_D$ et le temps t ;
    - calcul, pour chaque modèle, d'une probabilité de présence au temps t de l'analyte correspondant audit modèle à partir du calcul d'erreurs obtenu pour chaque capteur.

7.  Procédé de détection et d'identification selon la revendication 1, dans lequel un coefficient de confiance est attribué à chaque capteur C1, C2 et C3, chacun de ces coefficients reflétant la capacité dudit capteur à retransmettre, avec exactitude, l'adsorption ou la non adsorption d'un analyte sur le matériau sensible du capteur.

8.  Procédé de détection et d'identification selon la revendication 6 ou 7, dans lequel on calcule une différence, pour chaque capteur, entre une valeur de probabilité de présence calculée à un temps t et une valeur de probabilité de présence calculée à un temps t précédent pour ledit capteur et

    - si cette différence est inférieure, pour chaque capteur, à une valeur seuil P prédéterminée, alors on compare les valeurs de probabilité avec une valeur seuil D prédéterminée de probabilité et si la différence est supérieure à la valeur seuil prédéterminée P, alors l'analyte du modèle ayant la probabilité la plus importante correspond à l'analyte adsorbé sur le capteur, sinon la détection est considérée comme étant une erreur de détection ;
    - si la différence est supérieure à la valeur seuil D prédéterminée, alors on continue la comparaison.

9.  Procédé de détection et d'identification selon la revendication 1, dans lequel le transducteur optique est un transducteur à fluorescence, tandis que les transducteurs gravimétriques sont respectivement une microbalance à quartz et un transducteur à ondes acoustiques.

10. Procédé de détection et d'identification selon la revendication 1, dans lequel le détecteur comprend, outre les capteurs C1, C2 et C3, un ou plusieurs capteurs supplémentaires réalisés en plaçant un ou plusieurs matériaux sensibles supplémentaires sur un ou plusieurs des transducteurs des capteurs C1, C2 et C3.

11. Procédé de détection et d'identification selon la revendication 1, dans lequel la détection d'une variation du signal émis par l'un au moins de C1, C2 et C3 au-delà d'un seuil préalablement fixé pour le capteur émettant ce signal déclenche l'activation d'une alarme, sonore et/ou visuelle, qui avertit un utilisateur du détecteur de la présence d'un ou plusieurs analyte dans le milieu gazeux.

12. Procédé de détection et d'identification selon l'une quelconque des revendications 1 à 11, **caractérisé en ce qu'**il est utilisé pour détecter et identifier un ou plusieurs explosifs présent(s) dans un milieu gazeux.

13. Dispositif de détection et d'identification d'un analyte présent dans un milieu gazeux contenant un ou plusieurs analytes, qui comprend :

- un détecteur qui comprend au moins trois capteurs C1, C2 et C3, comprenant chacun un transducteur associé à un matériau sensible capable d'adsorber un ou plusieurs analytes,

C1 comprend un transducteur optique tandis que C2 et C3 comprennent des transducteurs gravimétriques différents l'un de l'autre,
chacun des capteurs C1, C2 et C3 émet un signal en l'absence de ce ou ces analytes,
et l'adsorption dudit ou desdits analytes par le matériau sensible d'un capteur produisant une variation du signal émis par ce capteur;
**caractérisé en ce que** le dispositif de détection et d'identification comprend aussi:

- des moyens d'acquisition et d'analyse adaptés à acquérir et analyser, de manière synchrone et répétitive, des signaux émis par C1, C2 et C3 pendant une mise en contact du détecteur avec le milieu gazeux, afin d'obtenir, pour chaque capteur, une courbe représentant le signal émis par ce capteur en fonction du temps, cette courbe représentant soit la ligne de base du capteur dans le cas où le matériau sensible de ce capteur n'adsorbe aucun des analytes, soit une cinétique d'adsorption qui est caractéristique du nombre d'analyte(s) adsorbé(s) par le matériau sensible du capteur et de ce(s) analyte(s) ;
- des moyens de détection adaptés à détecter une variation du signal émis par l'un au moins des capteurs C1, C2 et C3 au-delà d'au moins un seuil S, en valeur absolue, préalablement fixé pour le capteur émettant ce signal ;
- des moyens de comparaison adaptés à comparer la cinétique d'adsorption obtenue pour le capteur ayant une variation du signal émis, en valeur absolue, supérieure au seuil S, avec une pluralité de modèles de cinétiques d'adsorption préalablement établies pour chacun des analytes susceptibles d'être détectés par ce capteur.

**Patentansprüche**

1. Verfahren zum Nachweisen und Identifizieren eines Analyten, der in einem gasförmigen Medium vorhanden ist, das einen oder mehrere Analyten enthält, wobei
ein Detektor verwendet wird, der zumindest drei Sensoren C1, C2 und C3 enthält, die jeweils einen Wandler enthalten, der einem empfindlichen Material zugeordnet ist, das in der Lage ist, einen oder mehrere Analyten zu adsorbieren, C1 einen optischen Wandler enthält, während C2 und C3 gravimetrische Wandler enthalten, die sich voneinander unterscheiden,
jeder der Sensoren C1, C2 und C3 bei Nichtvorhandensein dieses bzw. dieser Analyten ein Signal ausgibt, und die Adsorption des/der Analyten durch das empfindliche Material eines Sensors eine Änderung des von diesem Sensor ausgegebenen Signals bewirkt;
wobei das Verfahren umfasst:

a) das Inkontaktbringen des Detektors mit dem gasförmigen Medium;

**dadurch gekennzeichnet, dass** das Verfahren ferner umfasst:
b) die synchrone und sich wiederholende Erfassung und Analyse der von C1, C2 und C3 ausgegebenen Signale während dieser Kontaktierung, um für jeden Sensor eine Kurve zu erhalten, die das von diesem Sensor ausgegebene Signal in Abhängigkeit von der Zeit darstellt, wobei diese Kurve entweder die Basislinie des Sensors für den Fall, dass das empfindliche Material dieses Sensors keinen der Analyten adsorbiert, oder eine Adsorptionskinetik darstellt, die für die Anzahl der von dem empfindlichen Material des Sensors adsorbierten Analyten und für diesen bzw. diese Analyten charakteristisch ist.;

wodurch dann,

wenn im Laufe von Schritt b) eine Änderung des von zumindest einen aus C1, C2 und C3 ausgegebenen Signals über zumindest einen Schwellenwert S im Absolutwert hinaus, der zuvor für den Sensor, der dieses Signal ausgibt, festgelegt wurde, erfasst wird, die Schritte a) und b) fortgesetzt werden und der bzw. die von dem empfindlichen Material dieses Sensors adsorbierten Analyten identifiziert wird bzw. werden, indem die für diesen Sensor erhaltene Adsorptionskinetik mit einer Vielzahl von adsorptionskinetischen Modellen verglichen wird, die zuvor für jeden der Analyten erstellt wurden, die von diesem Sensor erfassbar sind.

2. Verfahren zum Nachweisen und Identifizieren nach Anspruch 1, wobei die erfasste Änderung des Signals eine momentane Änderung zwischen zwei aufeinanderfolgenden Erfassungen des Sensors umfasst.

3. Verfahren zum Nachweisen und Identifizieren nach Anspruch 1 oder Anspruch 2, wobei die erfasste Änderung des Signals eine durchschnittliche Änderung über zumindest drei aufeinanderfolgende Erfassungen des Sensors umfasst.

4. Verfahren zum Nachweisen und Identifizieren nach Anspruch 2 oder Anspruch 3 in Kombination mit Anspruch 2, wobei das Erfassen einer Änderung eines Signals im Laufe von Schritt b) für jeden Sensor C1, C2 und C3 das Durchführen einer linearen Extrapolationsberechnung eines extrapolierten Wertes des von diesem Sensor ausgegebenen n-ten Signals ausgehend von den zuvor von diesem Sensor ausgegebenen (n - 1) Signalen umfasst, wobei n eine ganze Zahl größer oder gleich 3 ist, wobei die momentane Änderung die Differenz im Absolutwert zwischen dem extrapolierten Wert und dem erfassten Wert des von diesem Sensor ausgegebenen n-ten Signals ist, wobei die Erfassung des Signals berücksichtigt wird, wenn der Wert der momentanen Änderung im Absolutwert größer oder gleich einem zuvor festgelegten Schwellenwert S1 ist.

5. Verfahren zum Nachweisen und Identifizieren nach Anspruch 3 oder Anspruch 4 in Kombination mit Anspruch 3, wobei das Erfassen einer Änderung eines Signals im Laufe von Schritt b) für jeden Sensor C1, C2 und C3 das Durchführen einer Berechnung einer Steigung ausgehend von den zuvor von dem Sensor ausgegebenen (n - 1) Signalen umfasst, wobei n eine ganze Zahl größer oder gleich 3 ist, wobei der Absolutwert dieser Steigung der durchschnittlichen Änderung entspricht, wobei die Erfassung berücksichtigt wird, wenn der Wert der durchschnittlichen Änderung höher als ein vorgegebener Schwellenwert S2 ist

6. Verfahren zum Nachweisen und Identifizieren nach Anspruch 3, wobei die Identifizierung erhalten wird, indem die aufeinanderfolgenden nachstehenden Schritte ausgeführt werden:

- für jeden Sensor und für jedes Modell Berechnen einer Interpolationskurve gemäß dem Modell ausgehend von den Signalen des Sensors, die zwischen einer Zeit $t_D$, die der Zeit entspricht, bei der eine Änderung des von zumindest einem der Sensoren C1, C2 und C3 ausgegebenen Signals erfasst wird, und einer Zeit t erfasst werden;
- Berechnen von Fehlern für jeden Sensor und für jedes Modell zwischen der Interpolationskurve gemäß dem Modell und den Signalen des Sensors, die zwischen der Zeit $t_D$ und der Zeit t erfasst werden;
- für jedes Modell Berechnen einer Wahrscheinlichkeit des Vorhandenseins des Analyten zur Zeit t, der dem Modell entspricht, ausgehend von der erhaltenen Fehlerberechnung für jeden Sensor.

7. Verfahren zum Nachweisen und Identifizieren nach Anspruch 1, wobei jedem Sensor C1, C2 und C3 ein Vertrauenskoeffizient zugeordnet wird, wobei jeder dieser Koeffizienten die Fähigkeit des Sensors widerspiegelt, die Adsorption bzw. Nichtadsorption eines Analyten an dem empfindlichen Material des Sensors genau zu übertragen.

8. Verfahren zum Nachweisen und Identifizieren nach Anspruch 6 oder 7, wobei für jeden Sensor eine Differenz zwischen einem Wahrscheinlichkeitswert für das Vorhandensein, der zu einer Zeit t berechnet wird, und einem Wahrscheinlichkeitswert für das Vorhandensein, der zu einer früheren Zeit t für den Sensor berechnet wurde, berechnet wird und

- wenn diese Differenz geringer als ein vorbestimmter Schwellenwert P für jeden Sensor ist, die Wahrscheinlichkeitswerte mit einem vorbestimmten Schwellenwert D der Wahrscheinlichkeit verglichen werden, und wenn die Differenz höher als der vorbestimmte Schwellenwert P ist, der Analyt des Modells mit der höchsten Wahrscheinlichkeit dann dem am Sensor adsorbierten Analyten entspricht, wobei ansonsten die Erfassung als Erfassungsfehler betrachtet wird;
- wenn die Differenz höher als der vorbestimmte Schwellenwert D ist, das Vergleichen dann fortgesetzt wird.

9. Verfahren zum Nachweisen und Identifizieren nach Anspruch 1, wobei der optische Wandler ein Fluoreszenzwandler ist, während die gravimetrischen Wandler jeweils eine Quarzmikrowaage bzw. ein Schallwellenwandler sind.

10. Verfahren zum Nachweisen und Identifizieren nach Anspruch 1, wobei der Detektor neben den Sensoren C1, C2 und C3 einen oder mehrere zusätzliche Sensoren enthält, die durch Aufbringen von einem oder mehreren zusätzlichen empfindlichen Materialien auf einen oder mehrere der Wandler der Sensoren C1, C2 und C3 hergestellt sind.

11. Verfahren zum Nachweisen und Identifizieren nach Anspruch 1, wobei das Erfassen einer Änderung des von zumindest einem der Sensoren C1, C2 und C3 ausgegebenen Signals über einen vorab festgelegten Schwellenwert für den Sensor hinaus, der dieses Signal ausgibt, die Aktivierung eines akustischen und/oder visuellen Alarms auslöst, der einen Benutzer des Detektors auf das Vorhandensein eines oder mehrerer Analyten im gasförmigen Medium hinweist.

12. Verfahren zum Nachweisen und Identifizieren nach einem der Ansprüche 1 bis 11, **dadurch gekennzeichnet, dass** es zum Nachweisen und Identifizieren eines oder mehrerer explosiver Stoffe, die in einem gasförmigen Medium vorhanden sind, verwendet wird.

13. Vorrichtung zum Nachweisen und Identifizieren eines Analyten, der in einem gasförmigen Medium vorhanden ist, das einen oder mehrere Analyten enthält, enthaltend:

   - einen Detektor, der zumindest drei Sensoren C1, C2 und C3 enthält, die jeweils einen Wandler enthalten, der einem empfindlichen Material zugeordnet ist, das in der Lage ist, einen oder mehrere Analyten zu adsorbieren,

   wobei C1 einen optischen Wandler enthält, während C2 und C3 gravimetrische Wandler enthalten, die sich voneinander unterscheiden, wobei jeder der Sensoren C1, C2 und C3 bei Nichtvorhandensein dieses bzw. dieser Analyten ein Signal ausgibt,
   und die Adsorption des/der Analyten durch das empfindliche Material eines Sensors eine Änderung des von diesem Sensor ausgegebenen Signals bewirkt;
   **dadurch gekennzeichnet, dass** die Vorrichtung zum Nachweisen und Identifizieren auch enthält:

   - Erfassungs- und Analysemittel, die dazu geeignet sind, synchron und sich wiederholend von C1, C2 und C3 ausgegebene Signale während einer Kontaktierung des Detektors mit dem gasförmigen Medium zu erfassen und zu analysieren, um für jeden Sensor eine Kurve zu erhalten, die das von diesem Sensor ausgegebene Signal in Abhängigkeit von der Zeit darstellt, wobei diese Kurve entweder die Basislinie des Sensors für den Fall, dass das empfindliche Material dieses Sensors keinen der Analyten adsorbiert, oder eine Adsorptionskinetik darstellt, die für die Anzahl der von dem empfindlichen Material des Sensors adsorbierten Analyten und für diesen bzw. diese Analyten charakteristisch ist;
   - Erfassungsmittel, die dazu geeignet sind, eine Änderung des von zumindest einem der Sensoren C1, C2 und C3 ausgegebenen Signals über zumindest einen Schwellenwert S im Absolutwert hinaus zu erfassen, der zuvor für den Sensor, der dieses Signal ausgibt, festgelegt wurde;
   - Vergleichsmittel, die dazu geeignet sind, die für diesen Sensor erhaltene Adsorptionskinetik, die eine Änderung des ausgegebenen Signals aufweist, die im Absolutwert größer als der Schwellenwert S ist, mit einer Vielzahl von adsorptionskinetischen Modellen zu vergleichen, die zuvor für jeden der Analyten erstellt wurden, die von diesem Sensor erfassbar sind.

## Claims

1. Method for detecting and identifying an analyte present in a gaseous medium containing one or more analytes, wherein:

   a detector is used, which detector comprises at least three sensors C1, C2 and C3, each comprising a transducer associated with a sensing material capable of adsorbing one or more analytes,

C1 comprises an optical transducer whereas C2 and C3 comprise gravimetric transducers that are different from one another,
each of the sensors C1, C2 and C3 emits a signal in the absence of this or these analytes,
and the adsorption of said one or more analytes by the sensing material of a sensor producing a variation in the signal emitted by this sensor;
the method comprising the step of:

a) contacting the detector with the gaseous medium;

**characterised in that** the method further comprises the step of:
b) acquiring and analysing, synchronously and repetitively, the signals emitted by C1, C2 and C3 during this contacting in order to obtain, for each sensor, a curve representing the signal emitted by this sensor as a function of time, this curve representing either the baseline of the sensor in the case whereby the sensing material of this sensor does not adsorb any of the analytes, or adsorption kinetics which are characteristic of the number of the one or more analytes adsorbed by the sensing material of the sensor, and of this or these analytes; whereby:
if, during step b), a variation is detected in the signal emitted by at least one of C1, C2 and C3 beyond at least one threshold S, in absolute value form, previously determined for the sensor emitting this signal, steps a) and b) are continued and the one or more analytes adsorbed by the sensing material of this sensor are identified by comparing the adsorption kinetics obtained for this sensor with a plurality of adsorption kinetics models previously established for each of the analytes capable of being detected by this sensor.

2.  Detection and identification method according to claim 1, wherein the detected variation in the signal comprises an instant variation between two successive acquisitions of the sensor.

3.  Detection and identification method according to claim 1 or claim 2, wherein the detected variation in the signal comprises a mean variation over at least three successive acquisitions of the sensor.

4.  Detection and identification method according to claim 2 or according to claim 3 and claim 2 implemented together, wherein the detection of a variation in a signal during step b) comprises the performance, for each sensor C1, C2 and C3, of a linear extrapolation calculation of an extrapolated value of the $n^{th}$ signal emitted by this sensor from the (n-1) signals previously emitted by this sensor, n being an integer greater than or equal to 3, the instant variation being the difference, in absolute value form, between the extrapolated value and the acquired value of the $n^{th}$ signal emitted by said sensor, the detection of the signal being taken into account when the value, in absolute value form, of the instant variation is greater than or equal to a value of the previously determined threshold S1.

5.  Detection and identification method according to claim 3 or according to claim 4 and claim 3 implemented together, wherein the detection of a variation in a signal during step b) comprises the performance, for each sensor C1, C2 and C3, of a calculation of a slope from the (n-1) signals previously emitted by the sensor, n being an integer greater than or equal to 3, the absolute value of this slope corresponding to the mean variation, the detection being taken into account when the value of the mean variation is greater than a predetermined threshold value S2.

6.  Detection and identification method according to claim 3, wherein the identification is obtained by carrying out the successive steps of:

    - for each sensor and for each model, calculating an interpolation curve according to said model from the signals from said sensor acquired between a time $t_D$, corresponding to the time at which a signal variation emitted by at least one of the sensors C1, C2 and C3 is detected, and a time t;
    - calculating errors for each sensor and for each model between the interpolation curve according to said model and the signals from said sensor acquired between the time $t_D$ and the time t;
    - calculating, for each model, a probability of presence, at the time t, of the analyte corresponding to said model from the error calculation obtained for each sensor.

7.  Detection and identification method according to claim 1, wherein a confidence coefficient is assigned to each sensor C1, C2 and C3, each of these coefficients reflecting the capacity of said sensor to accurately retransmit the adsorption or non-adsorption of an analyte on the sensing material of the sensor.

8.  Detection and identification method according to claim 6 or 7, wherein a difference is calculated, for each sensor, between a probability of presence value at a time t and a probability of presence value calculated at an earlier time t for said sensor and

- if this difference is, for each sensor, less than a predetermined threshold value P, the probability values are compared with a predetermined probability threshold value D and if the difference is greater than the predetermined threshold value P, the analyte of the model having the highest probability corresponds to the analyte adsorbed on the sensor, otherwise the detection is considered to be a detection error;
- if the difference is greater than the predetermined threshold value D, the comparison is continued.

9. Detection and identification method according to claim 1, wherein the optical transducer is a fluorescence transducer, whereas the gravimetric transducers are a quartz crystal microbalance and an acoustic wave transducer respectively.

10. Detection and identification method according to claim 1, wherein the detector comprises, in addition to the sensors C1, C2 and C3, one or more additional sensors produced by placing one or more additional sensing materials on one or more of the transducers of the sensors C1, C2 and C3.

11. Detection and identification method according to claim 1, wherein the detection of a variation in the signal emitted by at least one of C1, C2 and C3 beyond a previously determined threshold for the sensor emitting this signal triggers the activation of an audible and/or visual alarm, which alerts the user of the detector to the presence of one or more analytes in the gaseous medium.

12. Detection and identification method according to any of claims 1 to 11, **characterised in that** it is used to detect and identify one or more explosives present in a gaseous medium.

13. Device for detecting and identifying an analyte present in a gaseous medium, containing one or more analytes, which device comprises:

- a detector which comprises at least three sensors C1, C2 and C3, each comprising a transducer associated with a sensing material capable of adsorbing one or more analytes,

C1 comprises an optical transducer whereas C2 and C3 comprise gravimetric transducers that are different from one another,
each of the sensors C1, C2 and C3 emits a signal in the absence of this or these analytes,
and the adsorption of said one or more analytes by the sensing material of a sensor producing a variation in the signal emitted by this sensor;
**characterised in that** the detection and identification device further comprises:

- acquisition and analysis means suitable for acquiring and analysing, synchronously and repetitively, the signals emitted by C1, C2 and C3 when the detector is contacting the gaseous medium, in order to obtain, for each sensor, a curve representing the signal emitted by this sensor as a function of time, this curve representing either the baseline of the sensor in the case whereby the sensing material of this sensor does not adsorb any of the analytes, or adsorption kinetics which are characteristic of the number of the one or more analytes adsorbed by the sensing material of the sensor and of this or these analytes;
- detection means suitable for detecting a variation in the signal emitted by at least one of the sensors C1, C2 and C3 beyond at least one threshold S, in absolute value form, previously determined for the sensor emitting this signal;
- comparison means suitable for comparing the adsorption kinetics obtained for the sensor having a variation in the signal emitted, in absolute value form, that is greater than the threshold S, with a plurality of adsorption kinetics models previously established for each of the analytes capable of being detected by this sensor.

Mise en route de la
détection

Acquisition en parallèle des
données des différents capteurs
à chaque temps
d'échantillonnage

Prédiction linéaire à un
pas sur une fenêtre
glissante

Non

Erreur de prédiction>$seuil_1$

Ou

abs(pente)>$seuil_2$

oui

Affichage
« Alarme »

Fusion de critères et de données des différents capteurs
pour déterminer la nature du (des) composé(s) chimiques
en présence dans l'atmosphère

Affichage du radar de
probabilité de détermination de
la nature de la menace

Suppression Alarme

Décision d'identification de la
nature du composé et du
type de menace

Non

Explosifs?

oui

Explosif(s) détecté(s)

FIG.1

## Multi Techno, EGDN

**FIG.2**

**FIG.3**

## Multi Techno, H2O2

FIG.4

FIG.5

Multi Techno, DNT

FIG.6

FIG.7

Multi Techno, TNT

FIG.8

FIG.9

24

## Multi Techno, MEK

FIG.10

FIG.11

## Multi Techno, DNT

FIG.12

FIG.13

Multi Techno, 4NT

FIG.14

FIG.15

FIG.16

FIG.17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2004259267 A1
- US 2004042933 A1
- US 2002178787 A1
- US 6955787 B1
- US 2004181346 A1
- US 2010259254 A1

- US 7446870 B2
- US 20080085212 A1
- US 6636811 B1
- WO 0016096 A1
- US 20060219892 A1
- FR 2868842

**Littérature non-brevet citée dans la description**

- **PENZA M et al.** Carbon nanotubes-coated multi-transducing sensors for VOCs detection. *SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS,* 2005, vol. 111-112, 171-180
- *CHEMICAL ABSTRACTS,* 261504-18-1
- **D. S. LEE et al.** Explosive gas recognition system using thick film sensor array and neural network. *Sensors and Actuators B: Chemical,* 2000, vol. 71, 90

- **J. PARK et al.** Vapor recognition with small arrays of polymer-coated microsensor. A comprehensive analysis. *Anal. Chem.,* 1999, vol. 71, 3877
- **M. BURL et al.** Classification performance of carbon black-polymer composite vapor detector arrays as a function of array size and detector composition. *Sensors and Acuators B: Chemical,* 2002, vol. 87, 130